# EUROPEAN PATENT SPECIFICATION

(11) **EP 3 737 372 B1**
(45) Date of publication and mention of the grant of the patent: **01.05.2024**
(21) Application number: 19729867.2
(22) Date of filing: 08.01.2019
(51) Int. Cl.: A61K 31/397, A61K 45/06, A61P 35/00, A61P 39/00

(54) **METHODS AND COMPOSITIONS UTILIZING RRX-001 FOR RADIOPROTECTION**
VERFAHREN UND ZUSAMMENSETZUNGEN MIT RRX-001 FÜR STRAHLENSCHUTZ
MÉTHODES ET COMPOSITIONS UTILISANT DU RRX-001 FOR LA RADIOPROTECTION

(30) Priority: 08.01.2018 US 201862614592 P; 26.09.2018 US 201862737093 P
(43) Date of publication of application: 18.11.2020
(73) Proprietor: EpicentRx, Inc., La Jolla, California 92037 (US)
(72) Inventor: ORONSKY, Bryan T., La Jolla, CA 92037 (US); ORONSKY, Arnold, La Jolla, CA 92037 (US)
(74) Representative: Hoffmann Eitle
(86) International application number: PCT/US2019/012701
(87) International publication number: WO 2019/164594

(56) References cited:
- WO-A2-2007/022225
- WO-A2-2013/052803
- BRYAN ORONSKY ET AL: "RRx-001: a systemically non-toxic M2-to-M1 macrophage stimulating and prosensitizing agent in Phase II clinical trials", EXPERT OPINION ON INVESTIGATIONAL DRUGS, vol. 26, no. 1, 21 December 2016 (2016-12-21), pages 109-119, XP55401288, UK ISSN: 1354-3784, DOI: 10.1080/13543784.2017.1268600
- S. NING ET AL: "Dinitroazetidines Are a Novel Class of Anticancer Agents and Hypoxia-Activated Radiation Sensitizers Developed from Highly Energetic Materials", CANCER RESEARCH, vol. 72, no. 10, 15 May 2012 (2012-05-15), pages 2600-2608, XP055615280, US ISSN: 0008-5472, DOI: 10.1158/0008-5472.CAN-11-2303
- JAN SCICINSKI ET AL: "NO to cancer: The complex and multifaceted role of nitric oxide and the epigenetic nitric oxide donor, RRx-001", REDOX BIOLOGY : AN OFFICIAL JOURNAL OF THE SOCIETY FOR FREE RADICAL BIOLOGY AND MEDICINE, AN OFFICIAL JOURNAL OF THE SOCIETY FOR FREE RADICAL RESEARCH-EUROPE, AN AFFILIATE JOURNAL OF THE INTERNATIONAL SOCIETY FOR FREE RADICAL RESEARCH (SFRRI), vol. 6, 1 December 2015 (2015-12-01), pages 1-8, XP55615083, NL ISSN: 2213-2317, DOI: 10.1016/j.redox.2015.07.002
- ORONSKY BRYAN ET AL: "RRx-001, A novel dinitroazetidine radiosensitizer", INVESTIGATIONAL NEW DRUGS, MARTINUS NIJHOFF PUBLISHERS, BOSTON, US, vol. 34, no. 3, 3 February 2016 (2016-02-03), pages 371-377, XP035906372, ISSN: 0167-6997, DOI: 10.1007/S10637-016-0326-Y [retrieved on 2016-02-03]
- KIM MICHELLE M ET AL: "Whole Brain Radiotherapy and RRx-001: Two Partial Responses in Radioresistant Melanoma Brain Metastases from a Phase I/II Clinical Trial: A TITE-CRM Phase I/II Clinical Trial.", TRANSLATIONAL ONCOLOGY APR 2016, vol. 9, no. 2, April 2016 (2016-04), pages 108-113, XP002793831, ISSN: 1936-5233
- "Safety and Efficacy of RRx-001 in the Attenuation of Oral Mucositis in Patients Receiving Chemoradiation for the Treatment of Oral Cancers (PREVLAR)", ClinicalTrials.gov , 3 May 2018 (2018-05-03), XP002793832, Retrieved from the Internet: URL:https://clinicaltrials.gov/ct2/show/NC T03515538 [retrieved on 2019-08-27]
- Eliot M. Rosen Et Al.: "New approaches to radiation protection", Frontiers in Oncology, 20 January 2015 (2015-01-20), pages 1-15, Retrieved from the Internet: URL:https://www.ncbi.nlm.nih.gov/pmc/artic les/PMC4299410/pdf/fonc-04-00381.pdf [retrieved on 2023-07-04]
- RAGHUNAND NATARAJAN ET AL: "Magnetic resonance imaging of RRx-001 pharmacodynamics in preclinical tumors", ONCOTARGET, vol. 8, no. 60, 24 November 2017 (2017-11-24), pages 102511-102520, DOI: 10.18632/oncotarget.18455
- BONOMI MARCELO ET AL: "PREVLAR: Phase 2a Randomized Trial to Assess the Safety and Efficacy of RRx-001 in the Attenuation of Oral Mucositis in Patients Receiving Head and Neck Chemoradiotherapy", INTERNATIONAL JOURNAL OF RADIATION: ONCOLOGY BIOLOGY PHYSICS, PERGAMON PRESS, USA, vol. 116, no. 3, 14 January 2023 (2023-01-14), pages 551-559, ISSN: 0360-3016, DOI: 10.1016/J.IJROBP.2022.12.031 [retrieved on 2023-01-14]

## Description

### PRIORITY

This application claims priority to U.S. Provisional Application Serial Nos. 62/614,592, filed January 8, 2018, and 62/737,093, filed September 26, 2018.

### STATEMENT OF FEDERAL FUNDING

This invention was made with government support under Grant No. RAB2436118 awarded by the U.S. Armed Forces Radiobiology Research Institute (AFRRI). The government has certain rights in the invention.

### FIELD OF THE INVENTION

Note that the references to methods of treatment in this description are to be interpreted as references to the compounds, pharmaceutical compositions and medicaments of the present invention for use in a method for treatment of the human (or animal) body by therapy.

Described are therapeutic methods, kits, and pharmaceutical compositions for protecting a subject from radiation using a therapeutic agent selected from the group consisting of 2-bromo-1-(3,3-dinitroazetidin-1-yl)ethan-1-one (RRx-001) and a pharmaceutically acceptable salt thereof, where an exemplary therapeutic method involves administering RRx-001 to the subject prior to the subject being exposed to the radiation, in order to protect the subject against radiation, such as ionizing radiation containing α-rays, β-rays, γ-rays, neutron radiation, or a combination thereof.

### BACKGROUND OF THE INVENTION

Ionizing radiation causes damage to normal tissues, ranging from genetic mutations to cell death. The harmful effects of ionizing radiation on normal tissues are a major concern for military and emergency responders to nuclear accidents and terrorist events due to the risk of acute and delayed radiation injuries. Additionally, radioprotection is a critical issue in cancer treatment. Despite significant technological improvements in radiation delivery in recent years, normal tissue toxicity remains a major dose-limiting factor in therapeutic radiology.

The development of safer and more effective radioprotection techniques is important for protecting civilians and military personnel from unintended radiation exposure. Such radiation may arise from nuclear power sources, nuclear emergencies, medical instrumentation that emits high levels of radiation, exposure to sunlight that has not been filtered through each of the earth's atmospheric layers, and from other sources. It is well known that radiation exposure can lead to cancer, such as, for example, leukemia. High-doses of radiation can also be lethal to humans and animal subjects. For these reasons, safer and more effective radioprotection techniques are needed.

RRx-001 (also called ABDNAZ), which has the chemical name 2-bromo-1-(3,3-dinitroazetidin-1-yl)ethan-1-one, is a small cyclic nitro compound that has previously been found to induce a number of enzymatic and epigenetic alterations in tumor cells. RRx-001 has been used clinically in combination with chemotherapy and/or radiation as a chemo- and radiosensitizer and is described in, for example, international patent application publication WO 2007/022225 describing various compounds and their use in treating medical disorders, such as cancer. Exemplary scientific publications describing benefits observed in human clinical trials evaluating efficacy of RRx-001 in treating patients suffering from cancer include Carter et al. in Respir. Med. Case Rep. (2016) vol. 18, pages 62-65; Kim et al. in Transl. Oncol. (2016) vol. 9(2), pages 108-113; and Reid et al. in Case Rep. Oncol. (2014) vol. 7(1), pages 79-85.

RRx-001 has been shown to have radioprotective properties when administered to the subject prior to exposure to the radiation (Bryan Oronsky et al. in Expert Opinion on Investigational Drugs (2016), vol.26 (1), pages 109-119; S. Ning et al. in Cancer Resaerch, (2012), vol.72(10), pages 2600-2608; Jan Scicinski et al. in Redox Biology, (2015), vol.6, pages 1-8).

### SUMMARY OF THE INVENTION

The present disclosure provides therapeutic methods, kits, and pharmaceutical compositions for protecting a subject from radiation using a therapeutic agent selected from the group consisting of RRx-001 and a pharmaceutically acceptable salt thereof, where an exemplary therapeutic method involves administering RRx-001 to the subject prior to the subject being exposed to the radiation, in order to protect the subject against radiation, such as ionizing radiation containing α-rays, β-rays, γ-rays, neutron radiation, or a combination thereof. The therapeutic methods have particular application in protecting civilians and military personnel from unintended radiation exposure, such as protecting first responders to a nuclear emergency, cosmic radiation associated with extended space habitat or travel, or other hazard involving harmful levels of radiation. In addition, the therapeutic methods can be employed in combination with radiation treatment for cancer for the protection of normal tissues. The therapeutic agent is desirably administered to the subject at least 6 hours, 12 hours, 24 hours, 36 hours, 48 hours, 3 days, 4 days, 5 days, 6 days, 1 week, 2 weeks, 3 weeks, or 4 weeks prior to the subject being exposed to radiation that could cause harm to the subject, and desirably provides protection against the harmful effects of radiation for a duration of at least 6 hours, 12 hours, 24 hours, 36 hours, 48 hours, 3 days, 4 days, 5 days, 6 days, 1 week, 2 weeks, 3 weeks, 4 weeks, or longer. The therapeutic agent is desirably administered to the subject through a procedure that minimizes pain experienced by the patient due to receiving RRx-001, such as by slow administration of RRx-001 or by administering RRx-001 after mixing with blood in order to reduce pain experienced by the patient. The invention having been generally described is explained in more detail in the aspects and embodiments below and in the detailed description.

Accordingly, one aspect of the disclosure provides a method for treating a subject in need of protection against radiation. The method comprises administering to the subject in need thereof an effective amount of a therapeutic agent selected from the group consisting of RRx-001 and a pharmaceutically acceptable salt thereof by a route selected from the group consisting of parenteral administration, oral administration, and topical administration, to thereby protect the subject against radiation for a duration of at least 6 hours, 12 hours, 24 hours, 36 hours, 48 hours, 3 days, 4 days, 5 days, 6 days, 1 week, 2 weeks, 3 weeks, 4 weeks, or longer. The therapeutic agent desirably is RRx-001, which desirably is administered to the subject by intravenous injection, intraperitoneal injection, subcutaneous injection, oral administration, or topical administration. At least one dose of the therapeutic agent is desirably administered to the subject prior to exposure to radiation.

Another aspect of the disclosure provides a method for reducing radiation-exposure damage to a subject. The method comprises administering to the subject in need thereof an effective amount of a therapeutic agent selected from the group consisting of RRx-001 and a pharmaceutically acceptable salt thereof by a route selected from the group consisting of parenteral administration, oral administration, and topical administration, to thereby reduce radiation-exposure damage to the subject for a duration of at least 6 hours. The therapeutic agent desirably is RRx-001, which desirably is administered to the subject by intravenous injection, intraperitoneal injection, subcutaneous injection, oral administration, or topical administration. At least one dose of the therapeutic agent is desirably administered to the subject prior to exposure to radiation.

Another aspect of the disclosure provides a method for protecting material, such as isolated cells, tissues or organs, from the damaging effects of radiation. The method comprises exposing the biological material to an effective amount of a therapeutic agent selected from the group consisting of RRx-001 and a pharmaceutically acceptable salt thereof, to thereby protect the biological material from the damaging effects of radiation for a duration of at least 6 hours, 12 hours, 24 hours, 36 hours, 48 hours, 3 days, 4 days, 5 days, 6 days, 1 week, 2 weeks, 3 weeks, 4 weeks, or longer. The therapeutic agent desirably is RRx-001. The biological material is desirably exposed to at least one dose of the therapeutic agent prior to exposure to the radiation.

Therapeutic agents described herein may be formulated as a pharmaceutical composition. One or more of the foregoing may be contained in a kit with instructions for use, as further described herein.

### BRIEF DESCRIPTION OF THE DRAWINGS

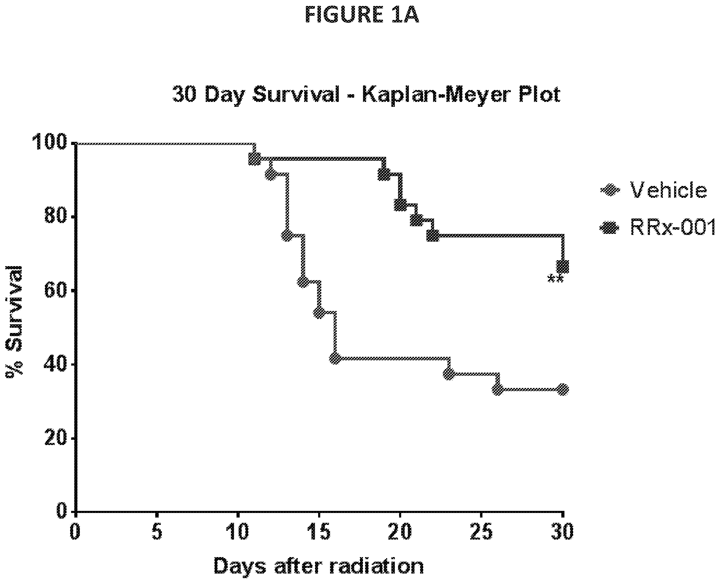

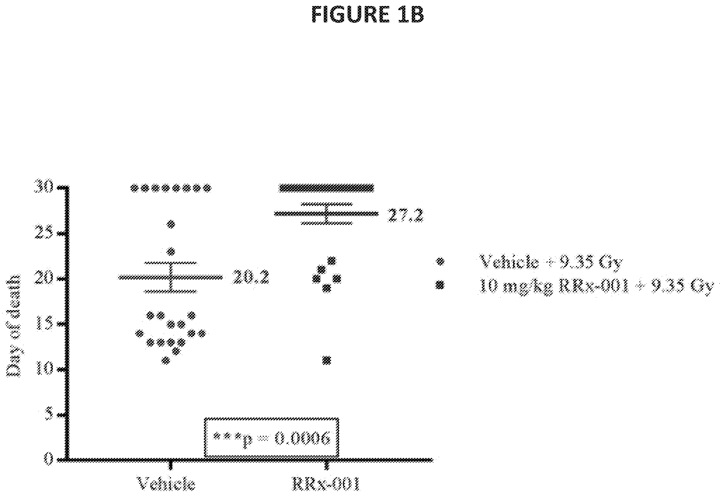

**FIG. 1A** illustrates the effects of RRx-001 treatment (10 mg/kg) on survival advantage following radiation (9.35 Gy (LD70/30) at 0.6 Gy/min) compared to an irradiated vehicle control. Data is presented as a 30 Day Survival Kaplan Meyer plot. N = 24/group; **p < 0.005. **FIG. 1B** provides a scatterplot of the survival times by treatment group. The means ± standard errors are 20.2 ± 1.6 and 27.2 ± 1.1 for vehicle and RRx-001 groups, respectively. ***p < 0.0006.

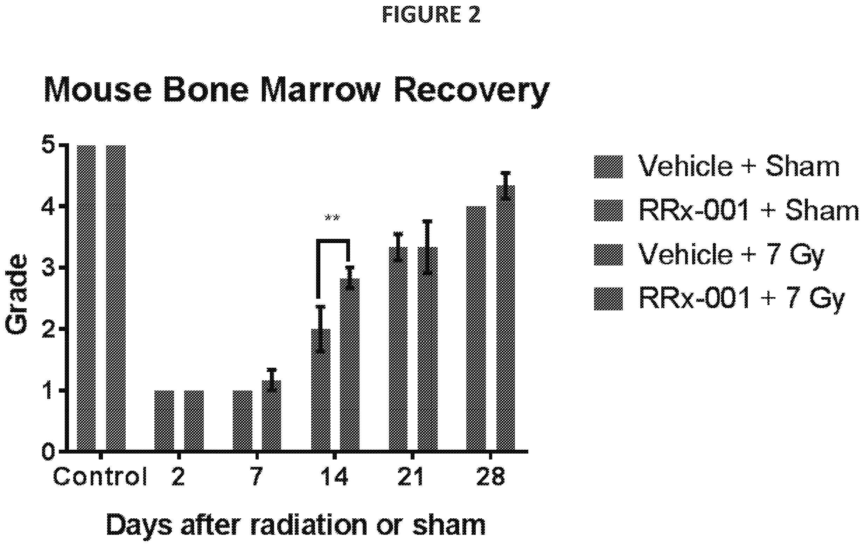

**FIG. 2** illustrates the effects of RRx-001 treatment on bone marrow recovery following sublethal dose of TBI (7 Gy at 0.6 Gy/min) compared to an irradiated vehicle control. Sham = No radiation control. N = 6/group/day; **p < 0.005; Error bars are mean ±SEM.

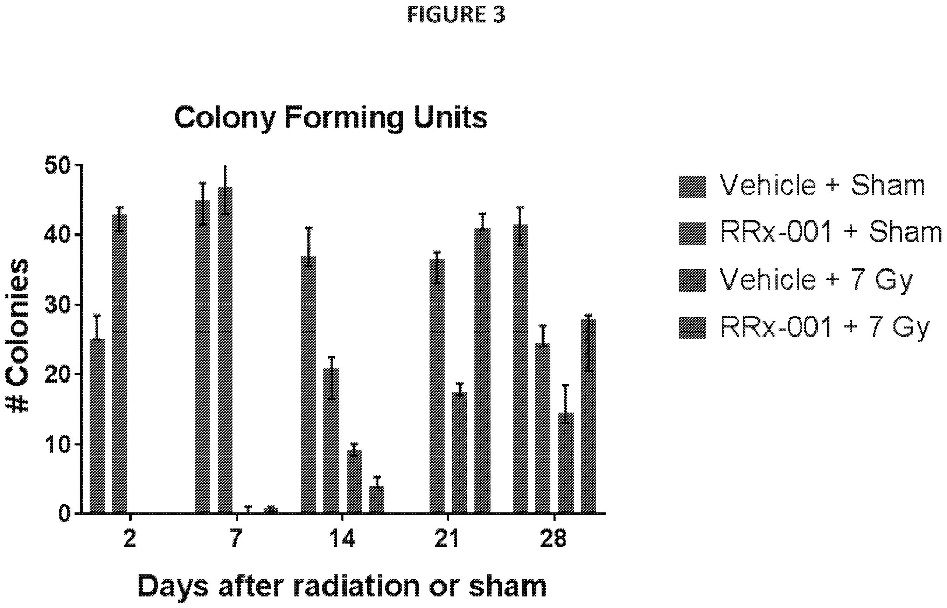

**FIG. 3** illustrates the effects of RRx-001 treatment on the number of colony forming units produced from mouse bone marrow following sublethal dose of TBI (7 Gy at 0.6 Gy/min) compared to an irradiated vehicle control. 3 mice/group/day were combined into one sample and plated in triplicate.

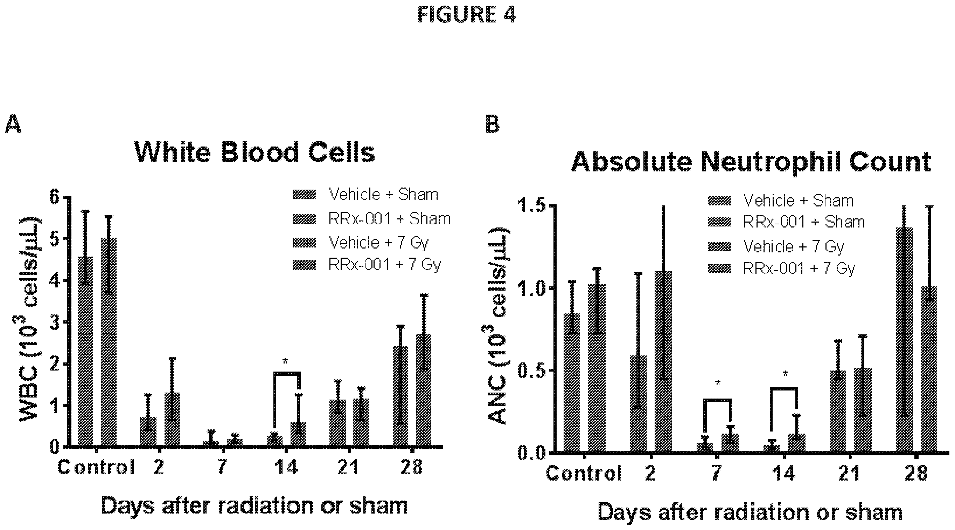

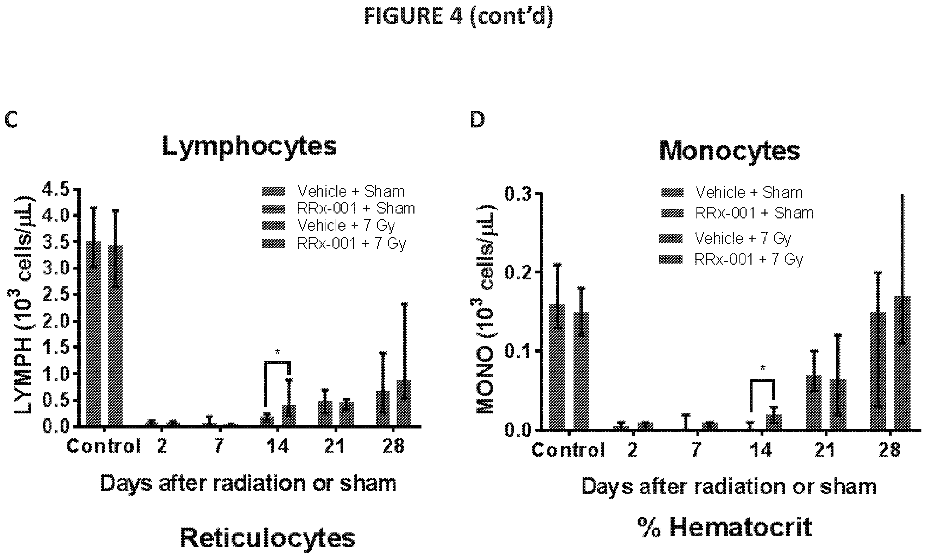

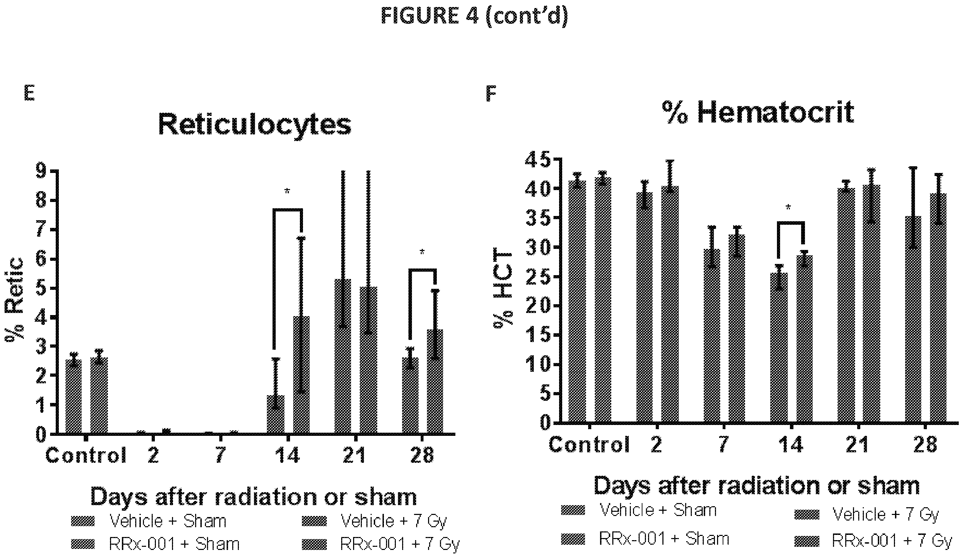

**FIG. 4** illustrates the radioprotective effects of RRx-001 treatment on blood cell production following sublethal dose of TBI (7 Gy at 0.6 Gy/min) compared to an irradiated vehicle control. Data is presented for white blood cell count **(****FIG. 4A****)**, absolute neutrophil count **(****FIG. 4B****)**, lymphocyte count **(****FIG. 4C****)**, monocyte count **(****FIG. 4D****)**, reticulocyte count **(****FIG. 4E****)**, and % hematocrit (percentage by volume of red cells to the volume of whole blood) **(****FIG. 4F****)**. N = 4 - 6 mice/group/day; *p < 0.05; Error bars are median with 95% CI.

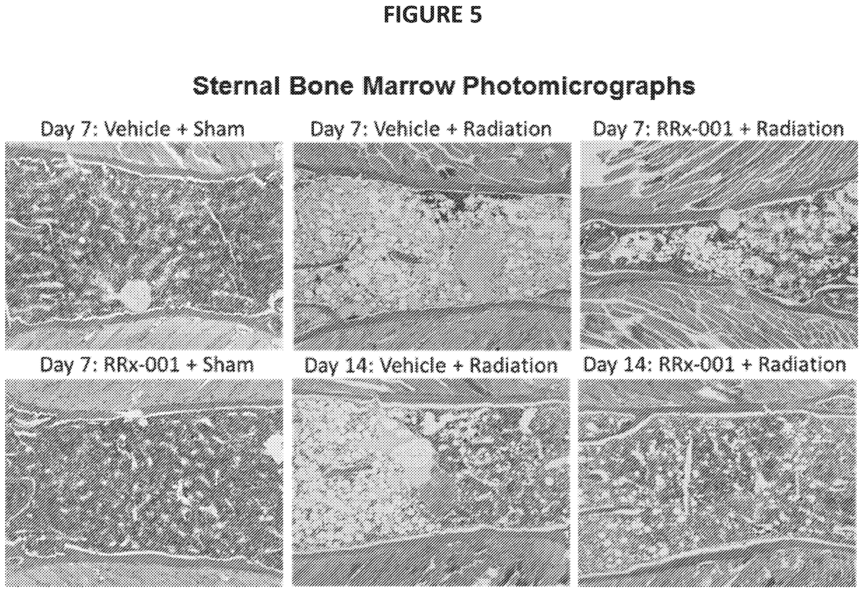

**FIG. 5** shows representative sternal bone marrow photomicrographs illustrating increased bone marrow recovery on days 7 and 14 in the RRx-001 + radiation group versus an irradiated vehicle control. All slides were stained with hematoxylin and eosin (H&E). Dark gray stain (center strip) is bone marrow (center gray horizontal strip), white is fat cells and light gray stain (top and bottom horizontal strips) is muscle.

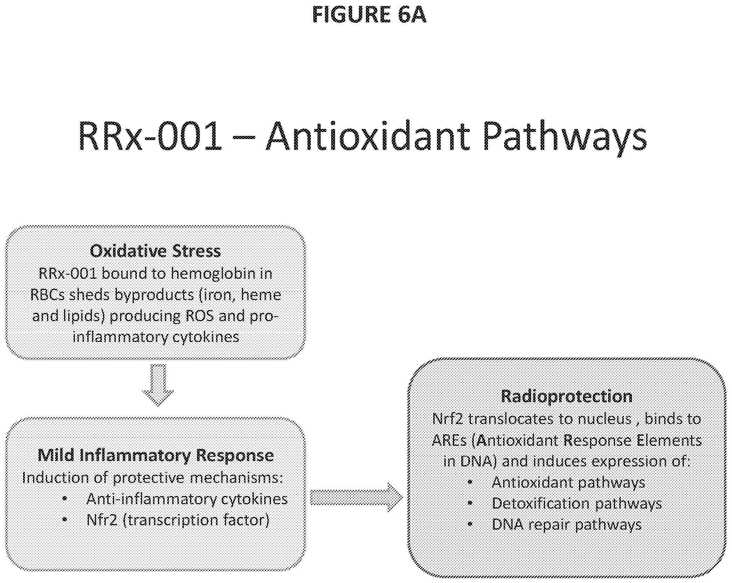

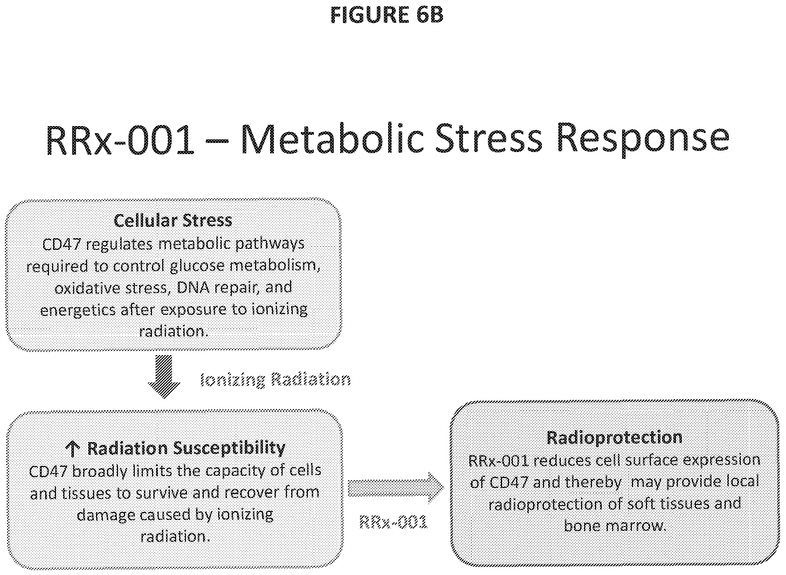

**FIG. 6** illustrates exemplary potential mechanisms for radioprotection by RRx-001 through antioxidant pathways **(****FIG. 6A****)** or the metabolic stress response **(****FIG. 6B****)**.

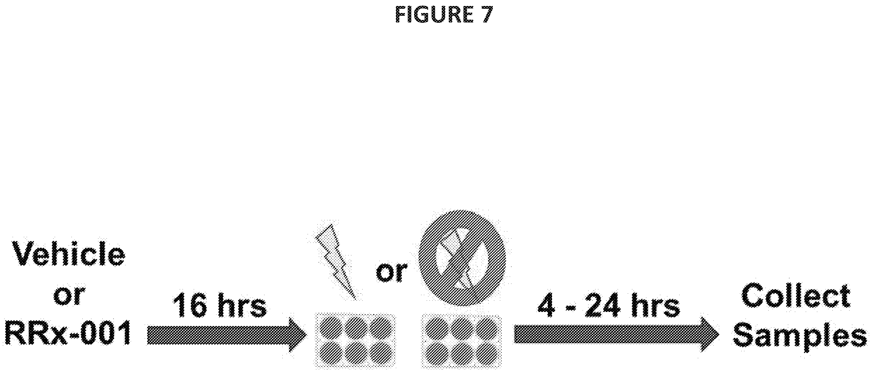

**FIG. 7** illustrates an *in vitro* model experimental design for characterizing the radioprotective effects of RRx-001.

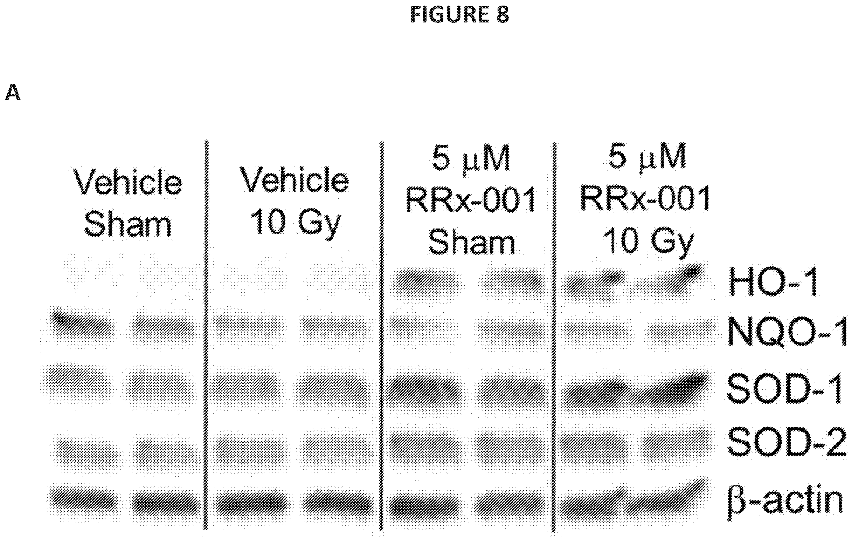

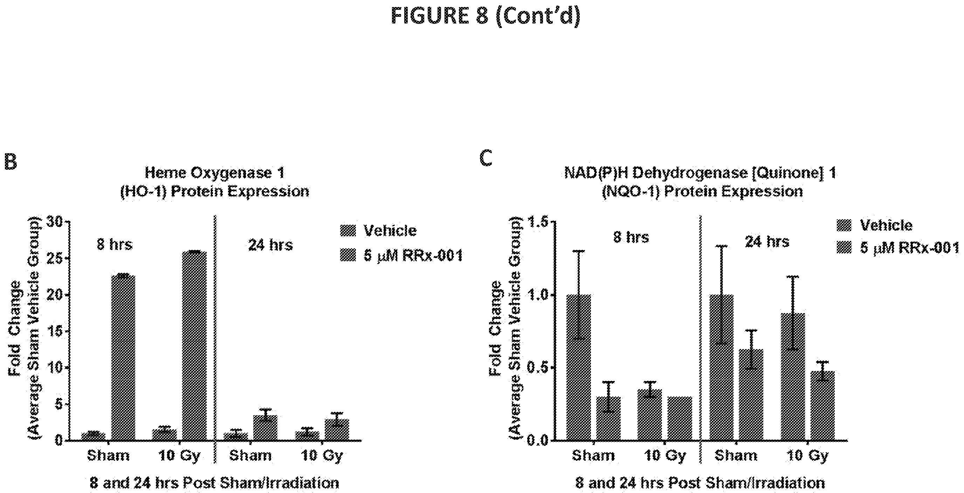

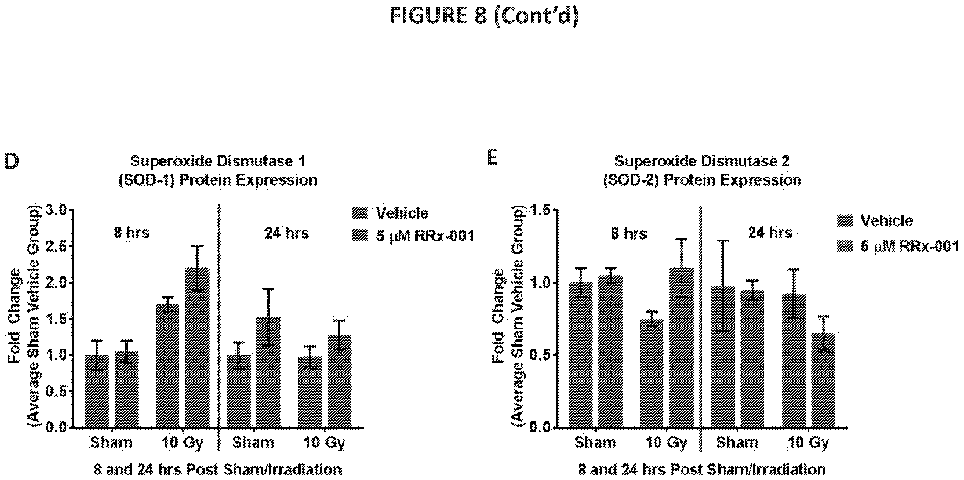

**FIG. 8** illustrates Western blotting **(****FIG. 8A****)** and quantification of protein expression (**FIGS. 8B-8E****)** of genes having antioxidant response elements (ARE) following irradiation of human mesenchymal stem cells treated with RRx-001 or a vehicle control. Data is shown for Heme oxengenase-1 (HO-1) **(****FIG. 8B****)**, quinine oxidoreductase- 1 (NQO-1) **(****FIG. 8C****)**, superoxide dismutase-1 (SOD-1) **(****FIG. 8D****)**, and superoxide dismutase-2 (SOD-2) **(****FIG. 8E****)**. Beta-actin expression was employed as a control.

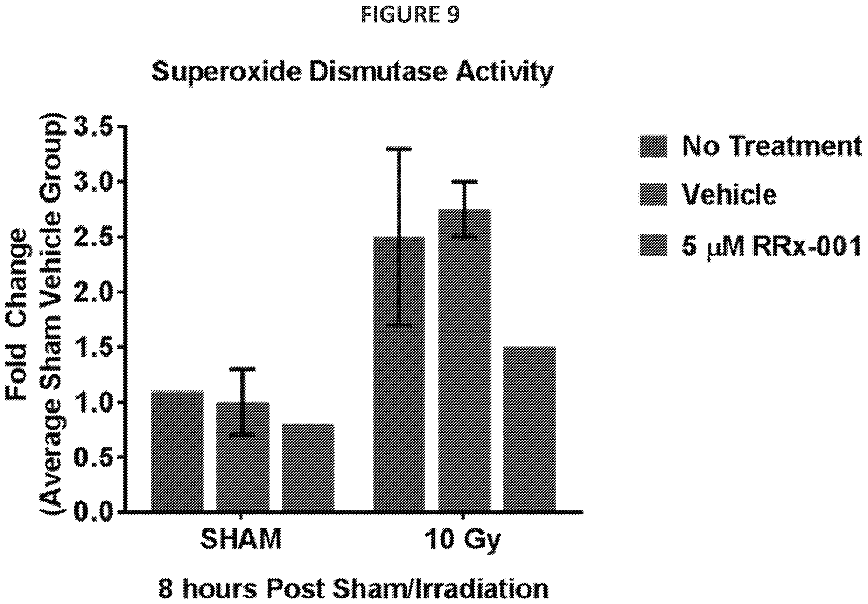

**FIG. 9** illustrates the effects of RRx-001 on superoxide dismutase activity in irradiated human mesenchymal stem cells compared to a vehicle control.

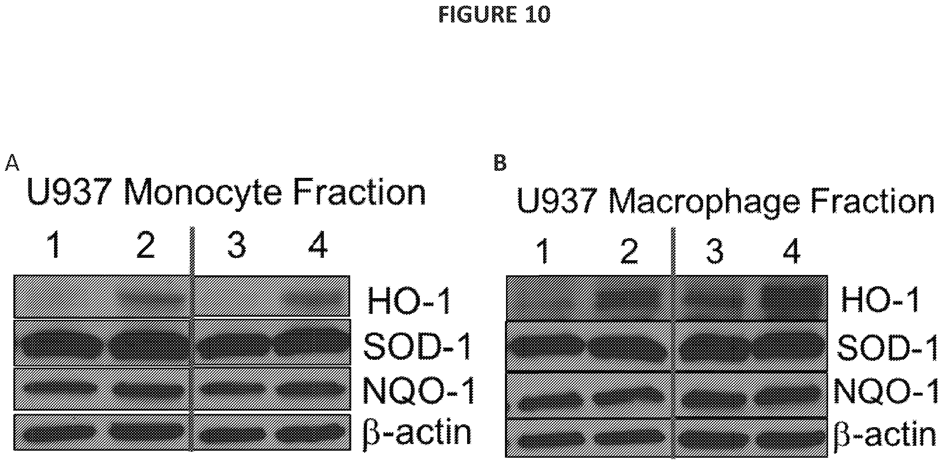

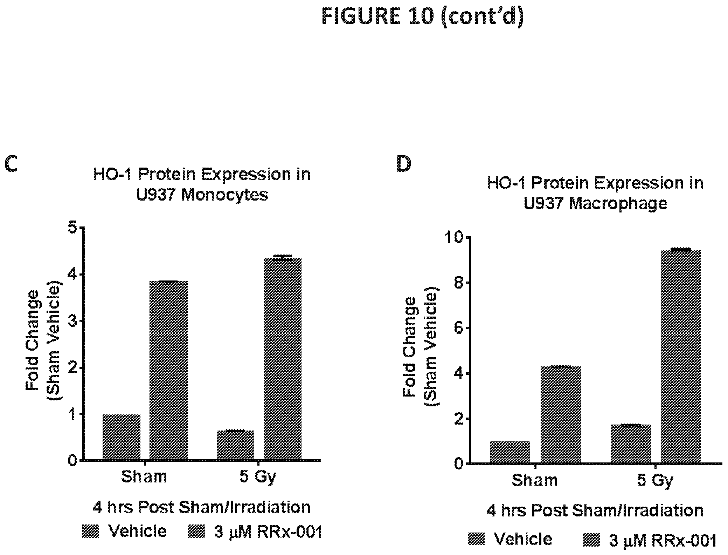

**FIGS. 10A and 10B** illustrate Western blots showing protein expression of HO-1, NQO-1, and SOD-1 in U937 monocyte fractions **(****FIG. 10A****)** and U937 macrophage fractions **(****FIG. 10B****)** 4 hours post-irradiation. Lane 1: vehicle/no irradiation; lane 2: vehicle/10 Gy irradiation; lane 3: RRx-001 (3 µM)/no irradiation; lane 4: RRx-001 (3 µM)/5 Gy irradiation. **FIGS. 10C-H** illustrate quantification of protein expression for each gene (HO-1, NQO-1, and SOD-1) expressed as fold change relative to the vehicle/no irradiation control U937 monocyte fractions and U937 macrophage fractions.

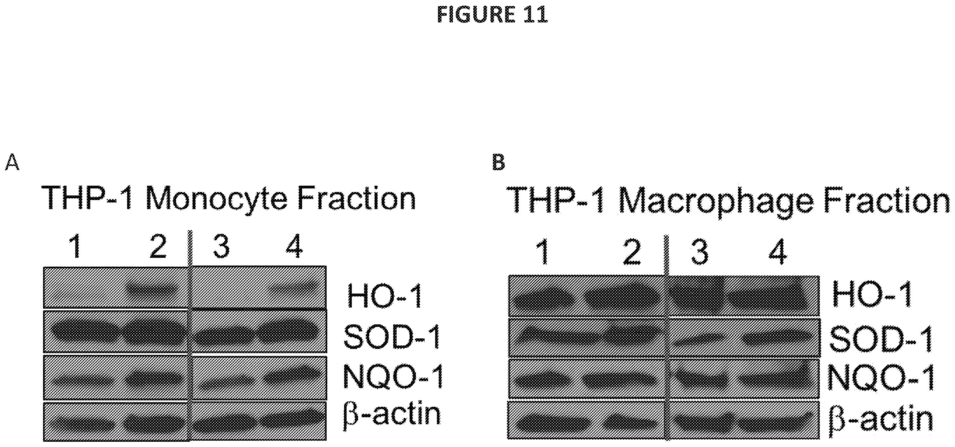

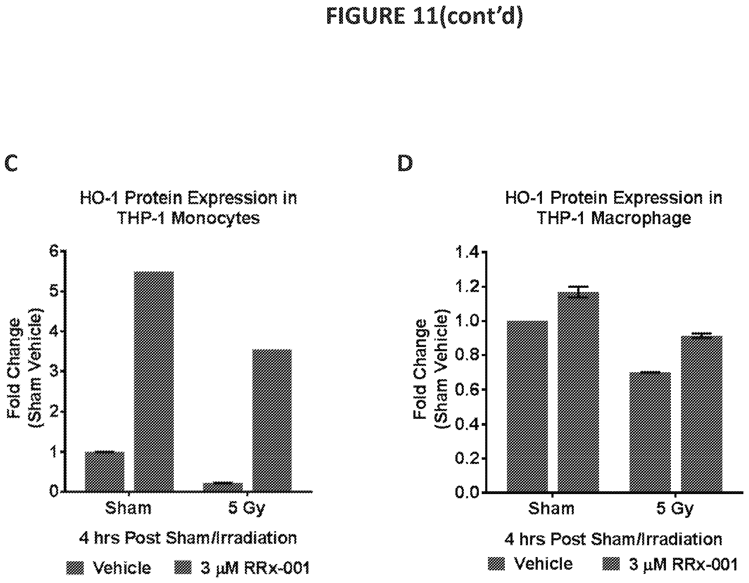

**FIGS. 11A and 11B** illustrate Western blots showing protein expression of HO-1, NQO-1, and SOD-1 in THP-1 monocyte fractions **(****FIG. 11A****)** and THP-1 macrophage fractions **(****FIG. 11B****)** 4 hours post-irradiation. Lane 1: vehicle/no irradiation; lane 2: vehicle/5 Gy irradiation; lane 3: RRx-001 (3 µM)/no irradiation; lane 4: RRx-001 (3 µM)/5 Gy irradiation. **FIGS. 11C-H** illustrate quantification of protein expression for each gene (HO-1, NQO-1, and SOD-1) expressed as fold change relative to the vehicle/no irradiation control in THP-1 monocyte fractions and THP-1 macrophage fractions.

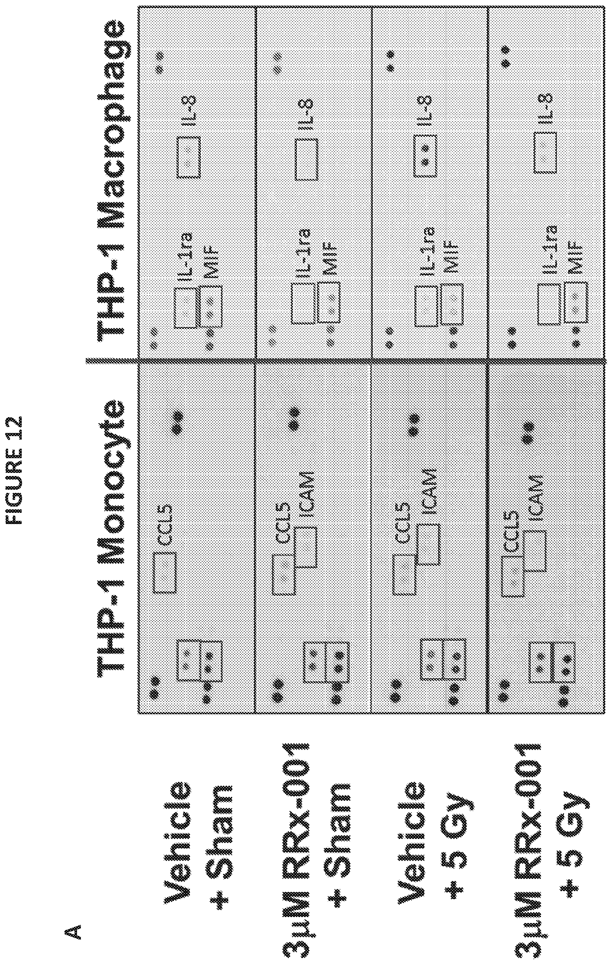

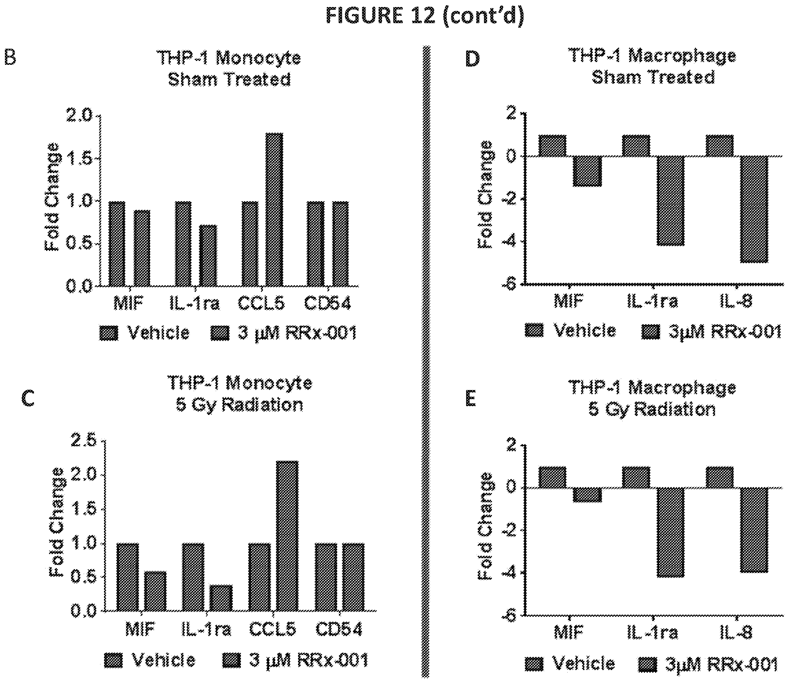

**FIG. 12A** illustrates dot blot expression of various cytokines and inflammatory modulators in irradiated THP-1 monocytes or THP-1 macrophages (5 Gy) treated with RRx-001 (3 µM) or vehicle control in THP-1 monocyte fractions and THP-1 macrophage fractions. **FIGS.12B-E** illustrate quantification of cytokine expression for selected genes expressed as fold change relative to the vehicle control in THP-1 monocyte fractions and THP-1 macrophage fractions.

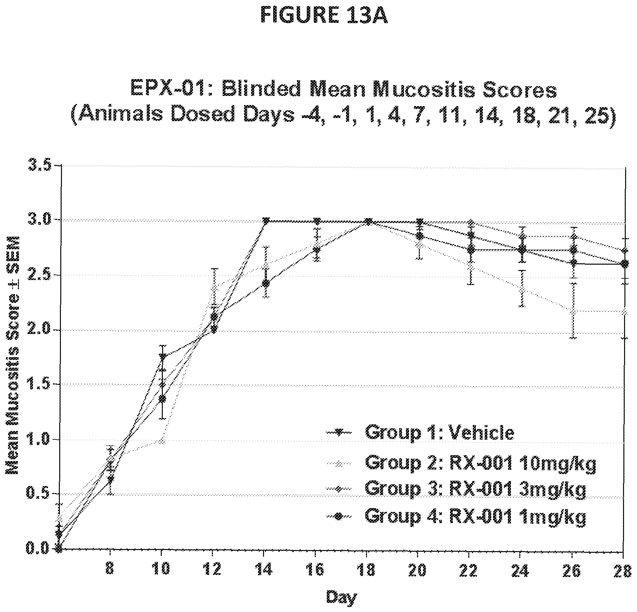

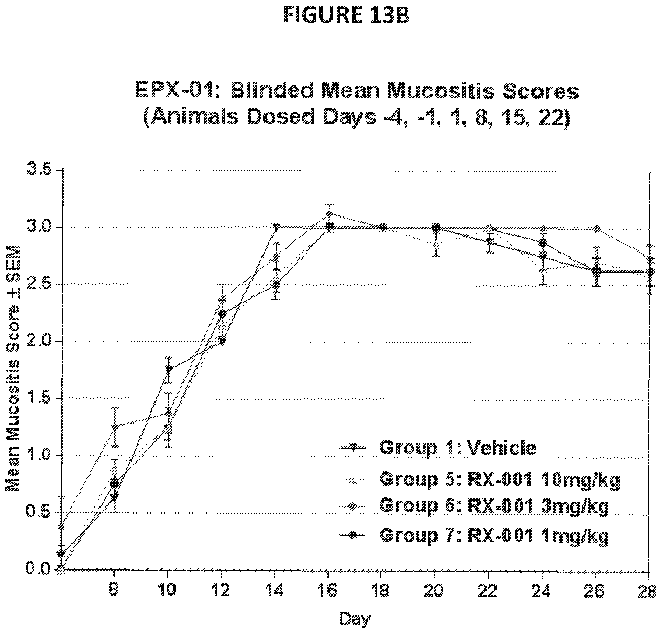

**FIG. 13** illustrates exemplary data for the effects of RRx-001 on alleviation of mucositis in a hamster model. **FIG. 13A** provides mean daily mucositis scores for the twice per week dosing groups. **FIG. 13B** provides mean daily mucositis scores for the once per week dosing groups. Mean group mucositis scores were calculated for each day of evaluation.

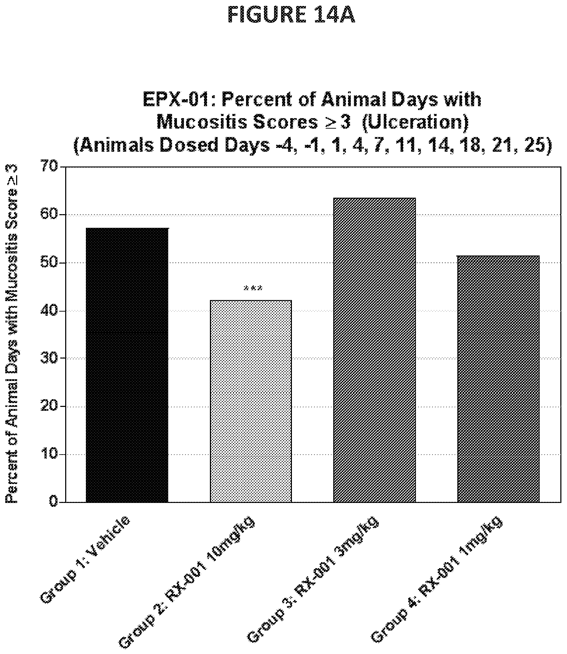

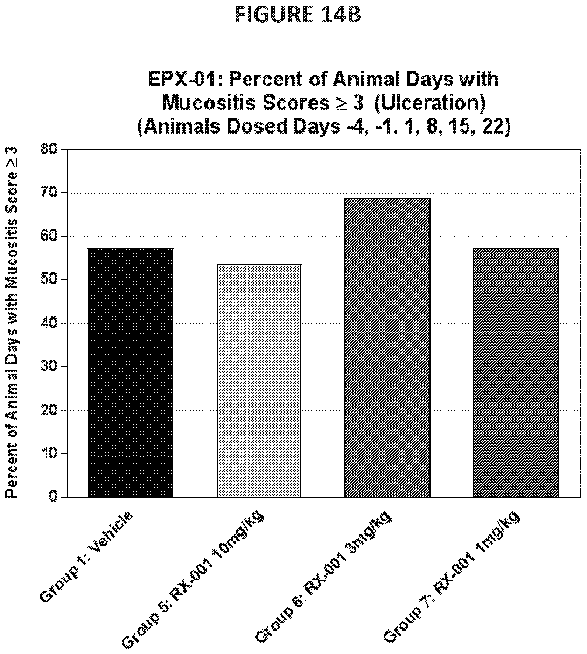

**FIG. 14** illustrates exemplary data for the effects of RRx-001 on alleviation of mucositis in a hamster model. **FIG. 14A** provides data for percent of days with Mucositis scores ≥ 3 for the entire study duration for the twice per week dosing groups. **FIG. 14B** provides data for percent of days with Mucositis scores ≥ 3 for the entire study duration for the once per week dosing groups. To examine the levels of clinically significant mucositis, as defined by presentation with open ulcers (a score of ≥ 3), the total number of days in which an animal exhibited an elevated score was summed and expressed as a percentage of the total number of days scored for the entire study duration (Day 6-28). Statistical significance was evaluated using the Chi-square test in comparison to Vehicle Control. ***p<0.001.

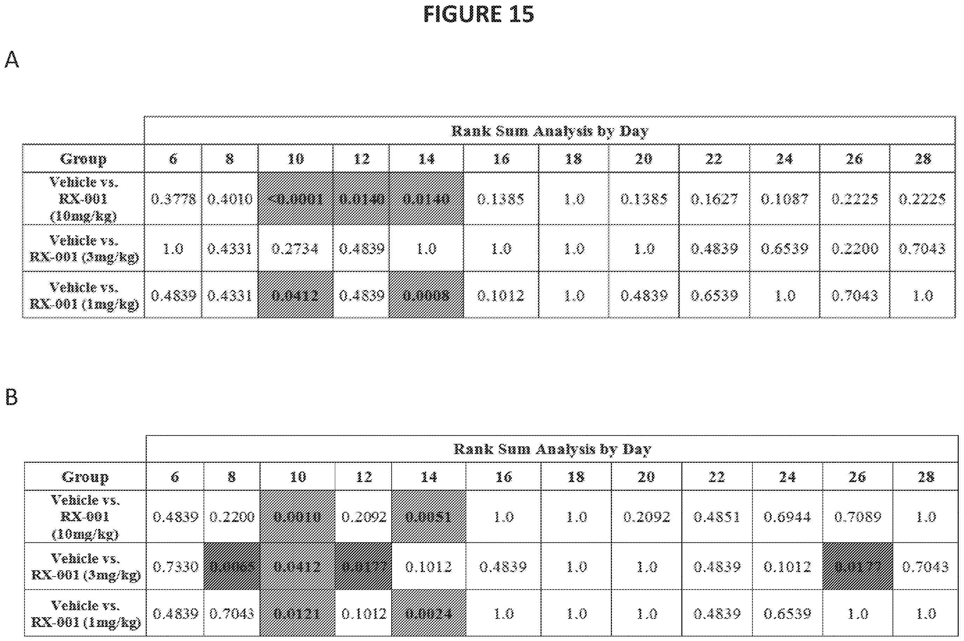

**FIG. 15** illustrates exemplary data for the comparison of daily mucositis scores (Groups Dosed -4, -1, 1, 4, 7, 11, 14, 18, 21, and 25). **FIG. 15A** provides data for the twice per week dosing groups. **FIG. 15B** provides data for the once per week dosing groups. The significance of group differences observed in daily mucositis scores was determined using the Mann-Whitney rank sum test. This nonparametric statistic is appropriate for the visual mucositis scoring scale. The p-values for each calculation are shown. Light grey shading denotes decrease in mucositis scores compared to Vehicle Group (improvement of disease), dark grey denotes increase in mucositis scores (worsening of disease). Bold font denotes significant difference in mucositis scores.

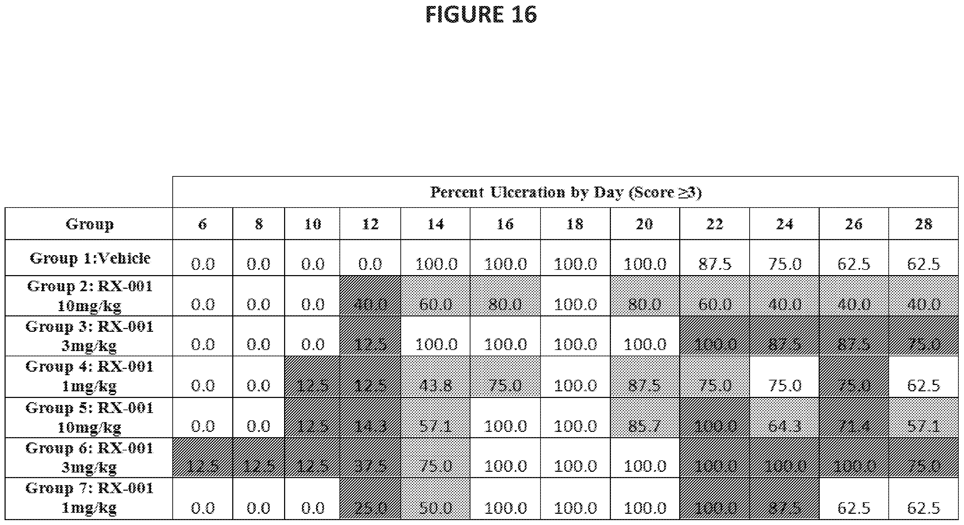

**FIG. 16** illustrates exemplary data for the percentages of animals with ulceration by day with a mucositis score ≥ 3. To examine the levels of clinically significant mucositis, as defined by presentation with open ulcers (score ≥ 3), the percentage of animals from each treatment group that exhibited an open ulcer on each day of the study was determined. Light shading denotes decrease in mucositis scores compared to Vehicle Group (improvement of disease), dark shading denotes increase in mucositis scores (worsening of disease).

### DETAILED DESCRIPTION OF THE INVENTION

### Definitions

Unless defined otherwise, all technical and scientific terms used herein have the meaning commonly understood by a person skilled in the art to which this disclosure belongs. The following references provide one of skill with a general definition of many of the terms used in this invention: Singleton et al., Dictionary of Microbiology and Molecular Biology (2nd ed. 1994); The Cambridge Dictionary of Science and Technology (Walker ed., 1988); The Glossary of Genetics, 5th Ed., R. Rieger et al., (eds.), Springer Verlag (1991); and Hale & Marham, The Harper Collins Dictionary of Biology (1991). As used herein, the following terms have the meanings ascribed to them below, unless specified otherwise. The terminology used herein is for the purpose of describing particular embodiments only and is not intended to be limiting of the disclosure.

The terms "a" and "an" as used herein mean "one or more" and include the plural unless the context is inappropriate.

As used herein, the terms "patient" and "subject" refer to organisms to be treated by the methods of the present disclosure. Such organisms are preferably mammals (*e*.*g*., marines, simians, equines, bovines, porcinis, canines, felines, and the like), and more preferably humans.

As used herein, the term "effective amount" refers to the amount of a compound (*e*.*g*., a compound of the present disclosure) sufficient to effect beneficial or desired results. An effective amount can be administered in one or more administrations, applications, or dosages and is not intended to be limited to a particular formulation or administration route.

As used herein, the term "treating" includes any effect, *e*.*g*., lessening, reducing, modulating, ameliorating or eliminating, that results in the improvement of the condition, disease, disorder, and the like, or ameliorating a symptom thereof.

As used herein, the terms "alleviate" and "alleviating" refer to reducing the severity of the condition, such as reducing the severity by, for example, at least 10%, 20%, 30%, 40%, 50%, 60%, 70%, 80%, 90%, or 95%.

As used herein, the term "pharmaceutical composition" refers to the combination of an active agent with a carrier, inert or active, making the composition especially suitable for diagnostic or therapeutic use *in vivo* or *ex vivo.*

As used herein, the term "pharmaceutically acceptable carrier" refers to any of the standard pharmaceutical carriers, such as a phosphate buffered saline solution, water, emulsions (*e*.*g*., such as an oil/water or water/oil emulsions), and various types of wetting agents. The compositions also can include stabilizers and preservatives. For examples of carriers, stabilizers and adjuvants, see, for example, Martin, Remington's Pharmaceutical Sciences, 15th Ed., Mack Publ. Co., Easton, PA [1975].

As used herein, the term "pharmaceutically acceptable salt" refers to any pharmaceutically acceptable salt (*e*.*g*., acid or base) of a compound of the present disclosure which, upon administration to a subject, is capable of providing a compound of this disclosure or an active metabolite or residue thereof. As is known to those of skill in the art, "salts" of the compounds of the present disclosure may be derived from inorganic or organic acids and bases. Examples of acids include, but are not limited to, hydrochloric, hydrobromic, sulfuric, nitric, perchloric, fumaric, maleic, phosphoric, glycolic, lactic, salicylic, succinic, toluene-p-sulfonic, tartaric, acetic, citric, methanesulfonic, ethanesulfonic, formic, benzoic, malonic, naphthalene-2-sulfonic, benzenesulfonic acid, and the like. Other acids, such as oxalic, while not in themselves pharmaceutically acceptable, may be employed in the preparation of salts useful as intermediates in obtaining the compounds of the disclosure and their pharmaceutically acceptable acid addition salts. Examples of bases include, but are not limited to, alkali metal (*e*.*g*., sodium) hydroxides, alkaline earth metal (*e*.*g*., magnesium) hydroxides, ammonia, and compounds of formula NW4+, wherein W is C1-4 alkyl, and the like.

Examples of salts include, but are not limited to: acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, flucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, palmoate, pectinate, persulfate, phenylpropionate, picrate, pivalate, propionate, succinate, tartrate, thiocyanate, tosylate, undecanoate, and the like. Other examples of salts include anions of the compounds of the present disclosure compounded with a suitable cation such as Na+, NH₄+, and NW₄+ (wherein W is a C1-4 alkyl group), and the like.

For therapeutic use, salts of the compounds of the present disclosure are contemplated as being pharmaceutically acceptable. However, salts of acids and bases that are non-pharmaceutically acceptable may also find use, for example, in the preparation or purification of a pharmaceutically acceptable compound.

The term "about" as used herein when referring to a measurable value (*e*.*g*., weight, time, and dose) is meant to encompass variations, such as ±10%, ±5% , ±1% , or ±0.1% of the specified value.

The compound RRx-001 (also called ABDNAZ) has the chemical name 2-bromo-1-(3,3-dinitroazetidin-1-yl)ethan-1-one, which has the following chemical structure:

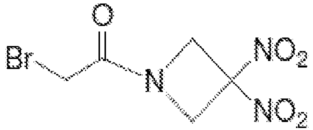

Throughout the description, where compositions are described as having, including, or comprising specific components, or where processes and methods are described as having, including, or comprising specific steps, it is contemplated that, additionally, there are compositions of the present disclosure that consist essentially of, or consist of, the recited components, and that there are processes and methods according to the present disclosure that consist essentially of, or consist of, the recited processing steps.

As a general matter, compositions specifying a percentage are by weight unless otherwise specified. Further, if a variable is not accompanied by a definition, then the previous definition of the variable controls

### Overview

Prophylactic radioprotective compounds that can protect normal tissue from the effects of ionizing radiation are an unmet need for military and first responders, space exploration, and cancer treatment. RRx-001 is a small cyclic nitro compound, 1-bromoacetyl-3,3-dinitroazetidine, which forms an RRx-001-hemoglobin adduct in red blood cells (RBC). RRx-001 is a systemically non-toxic anticancer agent that has been employed as a chemo- and radio-sensitizer for various tumors types in multiple clinical trials. In contrast with its ability to effect tumor radiosensitization, RRx-001 has been shown to protect normal cells from radiation (Scicinski et al., Redox Biology 2015;6:1). As described in the Examples provided herein, administration of RRx-001 prior to exposure to lethal radiation significantly increased survival in mice. Further, in sublethally irradiated mice, prophylactic administration of RRx-001 was found to significantly augment cellular recovery in bone marrow as evidenced by accelerated myeloreconstitution and improved bone marrow cellularity. In addition, RRx-001 treatment was found to increase expression of antioxidant response element proteins, such as heme oxygenase 1 (HO-1), in macrophages, monocytes, and mesenchymal stem cells. Induction of antioxidant response element genes may be driven by the transcription factor Nrf2, which has previously been shown to have increased nuclear presence in tumor cells exposed to RRx-001 (Ning et al., Oncotarget 2015;6(25):21547). Without wishing to be bound by theory, RRx-001 may provide cellular protection from oxidative injury via oxidative preconditioning whereby brief shifts in redox balance induce a precondition state of compensatory gene expression for antioxidant responses that are cytoprotective (see FIG. 6A). In addition, RRx-001 reduces the cell surface expression of the transmembrane protein CD47 (cluster differentiation 47), which may provide local radioprotection of soft tissues and bone marrow given that CD47 expression following exposure to ionizing radiation is known to limit the ability of cells and tissues to survive and recover from damage caused by ionizing radiation (Miller et al. (2015) J. Biol. Chem. 290: 24858-24874)(see FIG. 6B).

The present disclosure provides therapeutic methods, kits, and pharmaceutical compositions for protecting a subject from radiation using a therapeutic agent selected from the group consisting of RRx-001 and a pharmaceutically acceptable salt thereof.

In an exemplary therapeutic method, RRx-001 is administered to the subject prior to the subject being exposed to the radiation, in order to protect the subject against radiation, such as ionizing radiation containing α-rays, β-rays, γ-rays, neutron radiation or a combination thereof. The therapeutic methods have particular application in protecting civilians and military personnel from unintended radiation exposure, such as protecting first responders to a nuclear emergency, cosmic radiation associated with extended space habitat or travel, or other hazard involving harmful levels of radiation. In addition, the therapeutic methods can be employed in combination with radiation treatment for cancer for the protection of normal tissues. The therapeutic agent is administered to the subject at least 6 hours, 12 hours, 24 hours, 36 hours, 48 hours, 3 days, 4 days, 5 days, 6 days, 1 week, 2 weeks, 3 weeks, or 4 weeks prior to the subject being exposed to radiation that could cause harm to the subject, and desirably provides protection against the harmful effects of radiation for a duration of at least 6 hours, 12 hours, 24 hours, 36 hours, 48 hours, 3 days, 4 days, 5 days, 6 days, 1 week, 2 weeks, 3 weeks, 4 weeks, or longer.

### Methods For Protecting Against and Reducing Effects of Radiation

Provided herein are methods for protecting a subject from radiation using a therapeutic agent selected from the group consisting of RRx-001 and a pharmaceutically acceptable salt thereof. Various features of the methods are described in sections below. The sections are arranged for convenience and information in one section is not limited to that section, but may be applied to other sections.

One aspect of the present disclosure provides methods for treating a subject in need of protection against radiation. In some embodiments, the method comprises administering to the subject in need thereof an effective amount of a therapeutic agent selected from the group consisting of RRx-001 and a pharmaceutically acceptable salt thereof by a route selected from the group consisting of parenteral administration, oral administration, and topical administration, to thereby protect the subject against radiation for a duration of at least 6 hours, 12 hours, 24 hours, 36 hours, 48 hours, 3 days, 4 days, 5 days, 6 days, 1 week, 2 weeks, 3 weeks, 4 weeks, or longer. The therapeutic method has particular applications in protecting civilians and military personnel from unintended radiation exposure, such as protecting first responders to a nuclear emergency, cosmic radiation associated with extended space habitat or travel, or other hazard involving harmful levels of radiation. The therapeutic method can also be employed in combination with radiation therapy of a subject for cancer for the purpose of protecting the subject against radiation.

Another aspect of the present disclosure provides methods of reducing radiation-exposure damage to a subject. In some embodiments, the method comprises administering to the subject in need thereof an effective amount of a therapeutic agent selected from the group consisting of RRx-001 and a pharmaceutically acceptable salt thereof by a route selected from the group consisting of parenteral administration, oral and topical administration, to thereby reduce radiation-exposure damage to the subject for a duration of at least 6 hours, 12 hours, 24 hours, 36 hours, 48 hours, 3 days, 4 days, 5 days, 6 days, 1 week, 2 weeks, 3 weeks, 4 weeks, or longer. The therapeutic method has particular applications in protecting civilians and military personnel from unintended radiation exposure, such as protecting first responders to a nuclear emergency, cosmic radiation associated with extended space habitat or travel, or other hazard involving harmful levels of radiation. The therapeutic method can also be employed for reducing radiation-exposure damage associated with radiation therapy for cancer in a subject.

In some embodiments, administration of RRx-001 reduces or inhibits radiation-exposure damage to one or more cells, systems, organs, or normal tissues in a subject. In some embodiments, administration of RRx-001 reduces or inhibits radiation-exposure damage to one or more of bone marrow, lymphatic system, immune system, mucosal tissue, mucosal immune system, gastrointestinal system, cardiovascular system, nervous system, reproductive organs, prostate, ovaries, lung, kidney, skin and brain. In some embodiments, administration of RRx-001 reduces or inhibits one or more radiation-induced conditions, such as, but not limited to oral mucositis, dermatitis, skin rash, ulceration, alopecia, gastrointestinal distress, or proctitis.

Another aspect of the present disclosure provides methods of protecting biological material, such as isolated cells, tissues or organs, from the damaging effects of radiation. In some embodiments, the method comprises exposing said biological material to an effective amount of a therapeutic agent selected from the group consisting of RRx-001 and a pharmaceutically acceptable salt thereof, to thereby protect the biological material from the damaging effects of radiation for a duration of at least 6 hours, 12 hours, 24 hours, 36 hours, 48 hours, 3 days, 4 days, 5 days, 6 days, 1 week, 2 weeks, 3 weeks, 4 weeks, or longer.

Another (non-claimed) aspect of the present disclosure provides methods for treating a subject in need of protection against radiation using an agent that alkylates hemoglobin beta cysteine 93. In some embodiments, the agent that alkylates hemoglobin beta cysteine 93 is selected from the group consisting of RRx-001 and a pharmaceutically acceptable salt thereof. In some embodiments, the method comprises administering to the subject in need thereof an effective amount of a therapeutic agent that alkylates hemoglobin beta cysteine 93, to thereby protect the subject against radiation for a duration of at least 2 hours, 6 hours, 12 hours, 24 hours, 36 hours, 48 hours, 3 days, 4 days, 5 days, 6 days, 1 week, 2 weeks, 3 weeks, 4 weeks, or longer. In certain embodiments, the therapeutic agent is administered by a route selected from the group consisting of parenteral administration, oral administration, and topical administration. In certain embodiments, the method protects the subject against radiation for a duration of at least 6 hours. The therapeutic method has particular applications in protecting civilians and military personnel from unintended radiation exposure, such as protecting first responders to a nuclear emergency, cosmic radiation associated with extended space habitat or travel, or other hazard involving harmful levels of radiation. The therapeutic method can also be employed in combination with radiation therapy of a subject for cancer for the purpose of protecting the subject against radiation.

Another (non-claimed) aspect of the present disclosure provides a method of reducing radiation-exposure damage to a subject using an agent that alkylates hemoglobin beta cysteine 93. In some embodiments, the agent that alkylates hemoglobin beta cysteine 93 is selected from the group consisting of RRx-001 and a pharmaceutically acceptable salt thereof. The method comprises administering to the subject in need thereof an effective amount of a therapeutic agent that alkylates hemoglobin beta cysteine 93, to thereby reduce radiation-exposure damage to the subject for a duration of at least 2hours, 6 hours, 12 hours, 24 hours, 36 hours, 48 hours, 3 days, 4 days, 5 days, 6 days, 1 week, 2 weeks, 3 weeks, 4 weeks, or longer. In certain embodiments, the therapeutic agent is administered by a route selected from the group consisting of parenteral administration and topical administration. In certain embodiments, the method reduces radiation-exposure damage to the subject for a duration of at least 6 hours. The therapeutic method has particular applications in protecting civilians and military personnel from unintended radiation exposure, such as protecting first responders to a nuclear emergency, cosmic radiation associated with extended space habitat or travel, or other hazard involving harmful levels of radiation. The therapeutic method can also be employed for reducing radiation-exposure damage associated with radiation therapy for cancer in a subject.

In certain other (non-claimed) embodiments, the therapeutic agent that alkylates hemoglobin beta cysteine 93 comprises a maleimide. In certain embodiments, the therapeutic agent comprises an N-alkyl maleimide. In certain embodiments, the therapeutic agent comprises N-ethyl maleimide. In certain other embodiments, the therapeutic agent comprises a compound selected from the group consisting of an a-haloacetate, an a-haloacetamide, and an a-halomethylketone. In certain embodiments, the therapeutic agent is an a-haloacetate. In certain embodiments, the therapeutic agent comprises a-bromoacetate or a-iodoacetate. In certain other embodiments, the therapeutic agent comprises an a-haloacetamide. In certain embodiments, the therapeutic agent comprises a-bromoacetamide or a-iodoacetamide. In certain other embodiments, the therapeutic agent comprises an a-halomethylketone. In certain embodiments, the therapeutic agent comprises a-bromobenzophenone or a-iodobenzophenone. In certain embodiments, the therapeutic agent comprises a bromomethylketone. In certain embodiments, the therapeutic agent comprises an alpha-iodo-dinitroazetidine or an alpha-chloro-dinitroazetidine.

In certain embodiments, the methods provided herein achieve protection against radiation for a duration of at least 12 hours. In certain embodiments, the method achieves protection against radiation for a duration of at least 48 hours. In yet other embodiments, the method achieves protection against radiation for a duration of from about 6 hours to about 12 hours, from about 6 hours to about 24 hours, from about 12 hours to about 24 hours, or from about 24 hours to about 48 hours. In certain embodiments, the methods provided herein achieve protection against radiation for a duration of at least 1 week. In certain embodiments, the methods provided herein achieve protection against radiation for a duration of at least 1 month.

In certain embodiments, exemplary contemplated benefits of therapeutic methods may include, but are not limited to, (i) limiting the symptoms of acute radiation exposure, (ii) reducing the longer-term complications from radiation exposure, and/or (iii) prophylaxis against formation of cancers known to be caused by radiation exposure (for example, leukemias and thyroid cancers).

### Type and Source of the Radiation

The methods provided herein may be characterized according to the type of radiation. For example, in certain embodiments, the radiation is ionizing radiation. In certain embodiments, the radiation comprises α-rays, β-rays, γ-rays, neutron radiation, or a combination thereof. In certain other embodiments, the radiation comprises x-rays.

The method may also be characterized according to the source of the ionizing radiation. For example, in certain embodiments, the radiation is ionizing radiation from sunlight.

In certain other embodiments, the radiation is ionizing radiation from radioactive nuclei. In certain embodiments, the radiation is ionizing radiation from an explosive device.

In certain other embodiments, the radiation is from a medical device that emits therapeutic radiation, *e*.*g*. for the treatment of a cancer. Exemplary ionizing radiation treatment modalities can include, for example, external beam radiotherapy; Intensity modulated radiation therapy (INIRT); Image Guided Radiotherapy (IGRT); X Irradiation (*e*.*g*. photon beam therapy); electron beam (*e*.*g*. beta irradiation); local and total skin electron beam therapy; mega voltage photon treatment (about 4 to 10 MeV); proton irradiation; high linear energy transfer (LET) particles; stereotactic radiosurgery; gamma knife; linear accelerator mediated frameless stereotactic radiosurgery; robot arm controlled x irradiation delivery system; radioisotope radiotherapy for organ specific or cancer cell specific uptake; radioisotope bound to monoclonal antibody for tumor targeted radiotherapy (or radioimmunotherapy, RIT); brachytherapy (interstitial or intracavity) high dose rate radiation source implantation; permanent radioactive seed implantation for organ specific dose delivery.

### Methods for Administering the Therapeutic Agent

The therapeutic method may be characterized according to the timing for administering the therapeutic agent. For example, in certain embodiments, at least one dose of the therapeutic agent is administered to the subject prior to exposure to the radiation. According to the invention, at least one dose of the therapeutic agent is administered to the subject within at least 6 hours, 12 hours, 24 hours, 36 hours, 48 hours, 3 days, 4 days, 5 days, 6 days, 1 week, 2 weeks, 3 weeks, or 4 weeks prior to exposure to the radiation. In certain embodiments, at least one dose of the therapeutic agent is administered to the subject within 48 hours prior to exposure to the radiation. In certain embodiments, at least one dose of the therapeutic agent is administered to the subject within 24 hours prior to exposure to the radiation. In certain embodiments, at least one dose of the therapeutic agent is administered to the subject within 12 hours prior to exposure to the radiation. In certain embodiments, at least one dose of the therapeutic agent is administered to the subject at least 6 hours prior to exposure to the radiation.

In certain other embodiments, a dose of the therapeutic agent is first administered to the subject during exposure to the radiation or after exposure to the radiation has ceased. In non-claimed embodiments, a dose of the therapeutic agent is first administered to the subject during exposure to the radiation. In certain embodiments of the invention, a dose of the therapeutic agent is first administered to the subject after exposure to the radiation has ceased. In certain embodiments, a dose of the therapeutic agent is first administered to the subject within 1 day after exposure to the radiation has ceased. In certain embodiments, a dose of the therapeutic agent is first administered to the subject within 48, 24, 12, 6, 3, or 2 hours after exposure to the radiation has ceased. In certain embodiments, a dose of the therapeutic agent is first administered to the subject within 1 hour after exposure to the radiation has ceased.

In certain other embodiments, a dose of the therapeutic agent is administered to the subject (i) prior to exposure to the radiation and (ii) during exposure to the radiation. In certain other embodiments, a dose of the therapeutic agent is administered to the subject (i) prior to exposure to the radiation and (ii) after exposure to the radiation. In certain other embodiments, a dose of the therapeutic agent is administered to the subject (i) prior to exposure to the radiation, (ii) during exposure to the radiation, and (iii) after exposure to the radiation.

The therapeutic method may be characterized according to the frequency of administration of the therapeutic agent. For example, in certain embodiments, the therapeutic agent is administered to the subject no more frequently than once per week. In certain embodiments, the therapeutic agent is administered to the subject once per week for at least two weeks. In certain other embodiments, the therapeutic agent is administered to the subject at least once per week. In certain embodiments, the therapeutic agent is administered to the subject at least twice per week. In certain embodiments, the therapeutic agent is administered to the subject at least once per two days. In certain embodiments, the therapeutic agent is administered to the subject at least once per day. In certain embodiments, the therapeutic agent is administered to the subject at least twice per day.

The therapeutic method may be characterized according to the dose of the therapeutic agent. For example, in certain embodiments, the therapeutic agent is administered at a dosage that provides RRx-001 in an amount ranging from about 0.01 mg to about 1000 mg of RRx-001 on each day the therapeutic agent is administered to the subject. In certain embodiments, the therapeutic agent is administered at a dosage that provides RRx-001 in an amount ranging from about 0.05 mg to about 500 mg of RRx-001 on each day the therapeutic agent is administered to the subject. In certain embodiments, the therapeutic agent is administered at a dosage that provides RRx-001 in an amount ranging from about 0.1 mg to about 200 mg of RRx-001 on each day the therapeutic agent is administered to the subject. In certain embodiments, the therapeutic agent is administered at a dosage that provides RRx-001 in an amount ranging from about 0.5 mg to about 150 mg of RRx-001 on each day the therapeutic agent is administered to the subject. In certain embodiments, the therapeutic agent is administered at a dosage that provides RRx-001 in an amount ranging from about 1 mg to about 100 mg of RRx-001 on each day the therapeutic agent is administered to the subject. In certain embodiments, the therapeutic agent is administered at a dosage that provides RRx-001 in an amount ranging from about 5 mg to about 50 mg of RRx-001 on each day the therapeutic agent is administered to the subject. In certain embodiments, the therapeutic agent is administered at a dosage that provides RRx-001 in an amount ranging from about 0.5 mg to about 166 mg of RRx-001 on each day the therapeutic agent is administered to the subject.

In certain embodiments, the therapeutic agent is administered at a dosage that provides RRx-001 in an amount ranging from about 0.005 mg/m² to about 500 mg/m² of RRx-001 on each day the therapeutic agent is administered to the subject. In certain embodiments, the therapeutic agent is administered at a dosage that provides RRx-001 in an amount ranging from about 0.025 mg/m² to about 250 mg/m² of RRx-001 on each day the therapeutic agent is administered to the subject. In certain embodiments, the therapeutic agent is administered at a dosage that provides RRx-001 in an amount ranging from about 0.05 mg/m² to about 100 mg/m² of RRx-001 on each day the therapeutic agent is administered to the subject. In certain embodiments, the therapeutic agent is administered at a dosage that provides RRx-001 in an amount ranging from about 0.25 mg/m² to about 75 mg/m² of RRx-001 on each day the therapeutic agent is administered to the subject. In certain embodiments, the therapeutic agent is administered at a dosage that provides RRx-001 in an amount ranging from about 0.5 mg/m² to about 50 mg/m² of RRx-001 on each day the therapeutic agent is administered to the subject. In certain embodiments, the therapeutic agent is administered at a dosage that provides RRx-001 in an amount ranging from about 2.5 mg/m² to about 25 mg/m² of RRx-001 on each day the therapeutic agent is administered to the subject. In certain embodiments, the therapeutic agent is administered at a dosage that provides RRx-001 in an amount ranging from about 0.25 mg/m² to about 83 mg/m² of RRx-001 on each day the therapeutic agent is administered to the subject.

The therapeutic method may be characterized according to the route for administration of the therapeutic agent, and the method may be further characterized by the duration of administration. For example, in certain embodiments, the therapeutic agent is administered intravenously to the subject. In certain embodiments, the therapeutic agent is administered intravenously to the subject over a duration of at least thirty minutes. In certain embodiments, the therapeutic agent is administered intravenously to the subject over a duration of at least sixty minutes. In certain embodiments, the therapeutic agent is administered intravenously to the subject over a duration ranging from 30 minutes to 90 minutes.

In certain other embodiments, the therapeutic agent (e.g., RRx-001) is administered by intravenous injection of a mixture of blood with a composition comprising the therapeutic agent. In some embodiments, a quantity of blood (*e*.*g*., about 1 to about 50 mls of blood) is removed from the subject and mixed with a composition comprising the therapeutic agent (*e*.*g*., RRx-001). The mixture containing the therapeutic agent (*e*.*g*., RRx-001) is then administered intravenously to the patient. In some embodiments, the blood is mixed with an anticoagulant (*e*.*g*., using a syringe preload with an anticoagulant) prior to mixing with the therapeutic agent. In some embodiments, the method of removing the blood from the subject, mixing with the therapeutic agent and administering the mixture to the subject are performed in an aseptic closed system (*e*.*g*., connected system of sterile tubing, syringes, containers, and the like), where the blood is not exposed to the environment. In some embodiments, the closed system is flushed with sterile saline where the saline flush is administered to the patient to ensure complete delivery of the therapeutic agent.

In certain other embodiments, the therapeutic agent is administered by intraperitoneal injection to the subject. In certain embodiments, the therapeutic agent is administered by intraperitoneal injection to the subject over a duration of at least thirty minutes.

In certain other embodiments, the therapeutic agent is administered by subcutaneous injection. In certain embodiments, the therapeutic agent is administered by subcutaneous injection to the subject over a duration of at least 5 minutes. In certain other embodiments, the therapeutic agent is administered subcutaneously to the subject via a pump device implanted in the subject that contains the therapeutic agent. In certain embodiments, when the therapeutic agent is administered subcutaneously to the subject via a pump device implanted in the subject that contains the therapeutic agent, the pump device is an osmotic pump.

In certain other embodiments, the therapeutic agent is administered by topical administration. The topical administration may be, for example, a topical gel containing the first therapeutic agent, which is applied to the skin of the subject. The topical gel may be a sustained release gel that slowly releases the first therapeutic agent over time.

In certain other embodiments, the therapeutic agent is administered by oral administration, such as a pill, a capsule, sublingual tablets, sustained-release formulation, delayed-release formulation, a liquid, or an aerosol.

The therapeutic method may be characterized according to the location for administration of the therapeutic agent. For example, in certain embodiments, the therapeutic agent is administered in proximity to tissue desired to be protected from radiation. In certain embodiments, tissue desired to be protected from radiation is bone marrow, skin, pulmonary tissue, thyroid tissue, gonadal tissue, tissue of the gastrointestinal tract, skeletal tissue, fetal tissue, or a combination thereof.

### Subjects for Treatment

The therapeutic method may be further characterized according to the subject to be treated. In certain embodiments, the subject is a human. In certain embodiments, the subject is an adult human. In certain embodiments, the subject is an adult human at risk of exposure to radiation from a nuclear emergency. In certain other embodiments, the subject is a pediatric human. In certain other embodiments, the subject is an animal, such as a domesticated animal (*e*.*g*., a dog, a cat, or livestock).

In certain other embodiments, the subject is at risk of exposure to radiation from a nuclear emergency or from space travel. In certain embodiments, the subject is at risk of exposure to radiation from a nuclear emergency. In certain other embodiments, the subject is at risk of exposure to radiation from space travel. In certain other embodiments, the subject is an astronaut.

In certain other embodiments, the subject is at risk of radiation induce damage due radiation therapy for the treatment of a cancer.

In certain other embodiments, the subject has a suppressed immune system. In certain embodiments, the suppressed immune system is caused by an immunosuppressive medication. In certain embodiments, the immunosuppressive medication is a steroid, a calcineurin inhibitor, an interleukin-receptor-inhibiting antibody, or an interferon. In certain embodiments, the immunosuppressive medication is a steroid. In certain other embodiments, the suppressed immune system is caused by an immune deficiency syndrome (*e*.*g*., human immunodeficiency virus). In certain other embodiments, the subject having a suppressed immune system is a subject that has a history of hematopoietic stem cell transplantation in order to help ameliorate the symptoms of a suppressed immune system.

### Administration of Additional Therapeutic Agents

In certain embodiments, the methods provided herein further comprise administering the therapeutic agent (*e*.*g*., RRx-001 or a pharmaceutically acceptable salt thereof) in combination with one or more additional therapeutic agents. In some embodiments, the one or more additional therapeutic agents is administered prior to, concurrently, or subsequent to administration of the therapeutic agent (*e*.*g*., RRx-001 or a pharmaceutically acceptable salt thereof).

### Pain-Relieving Agent

In certain embodiments, the methods provided herein further comprise administering the therapeutic agent (*e*.*g*., RRx-001 or a pharmaceutically acceptable salt thereof) in combination with a pain-relieving agent. In some embodiments, the pain-relieving agent is administered prior to, concurrently, or subsequent to administration of the therapeutic agent (*e*.*g*., RRx-001 or a pharmaceutically acceptable salt thereof). Exemplary pain relieving agents include a local analgesic, aspirin, a corticosteroid, and non-steroidal anti-inflammatory agent. In certain embodiments, the pain-relieving agent is aspirin, a corticosteroid, or a nonsteroidal anti-inflammatory agent.

In certain embodiments, the method further comprises, prior to administration of the therapeutic agent, administering to the subject a local analgesic agent to tissue in proximity to the site of administration of the therapeutic agent. In certain embodiments, the local analgesic agent is a caine analgesic. In certain embodiments, the local analgesic agent comprises lidocaine. In certain embodiments, the local analgesic agent is lidocaine hydrochloride. In certain other embodiments, the local analgesic agent is VanPen cream. In certain other embodiments, the local analgesic agent is a NSAID. In certain other embodiments, the local analgesic agent is acetaminophen. In certain other embodiments, the local analgesic agent is VanPen cream, a NSAID, or acetaminophen.

In certain embodiments, the local analgesic agent is a formulation that comprises: i) a single active ingredient selected from the group consisting of lecithin, isopropyl palmitate, isopropyl myristate, and combinations thereof; and ii) excipients to form an ointment, cream, gel, lotion, spray, foam, paste, suspension or dispersion, for topical application to the skin. In certain embodiments, the single active ingredient is a combination of lecithin, isopropyl palmitate, and isopropyl myristate. In certain embodiments, formulation comprises soya lecithin, isopropyl palmitate, steric acid, glycerol, monostearate, isopropyl myristate, and polyoxyl 40 stearate.

In certain embodiments, the local analgesic agent is a formulation that consists of (i) lecithin and optionally one or two penetration enhancer fatty acid ester compounds, as the only active ingredients, and (ii) excipients to form an ointment, cream, gel, lotion, spray, foam, paste, suspension or dispersion for topical application to the skin of the subject. In certain embodiments, the formulation has one or two penetration enhancer fatty acid ester compounds.

In certain embodiments, the formulation has one penetration enhancer fatty acid ester compound selected from the group consisting of isopropyl palmitate and isopropyl laurate. In certain embodiments, the penetration enhancer fatty acid ester compound is isopropyl palmitate. In certain embodiments, one of the excipients is an emulsifier. In certain embodiments, the emulsifier is a poloxamer, polyoxyethylene alkyl ether, polyoxyethylene sorbitan fatty acid ester, or polyoxyethylene stearate. In certain embodiments, the emulsifier is a polyoxyethylene stearate. In certain embodiments, another one of the excipients is a surfactant selected from the group consisting of glycerin monostearate and glyceryl monooleate. In certain embodiments, the formulation is in the form of a gel.

### Anticancer Agent

In certain embodiments, the methods further comprise administering an anticancer agent to the subject in combination with the therapeutic agent (*e*.*g*., RRx-001 or a pharmaceutically acceptable salt thereof). In certain embodiments, the anticancer agent is a chemotherapeutic agent (also referred to as an anti-neoplastic agent or anti-proliferative agent). Exemplary chemotherapeutic agents include, but are not limited to, an alkylating agent, an antibiotic, an anti-metabolite, a detoxifying agent, an interferon, a polyclonal or monoclonal antibody, an EGFR inhibitor, a HER2 inhibitor, a histone deacetylase inhibitor, a hormone, a mitotic inhibitor, an MTOR inhibitor, a multi-kinase inhibitor, a serine/threonine kinase inhibitor, a tyrosine kinase inhibitors, a VEGF/VEGFR inhibitor, a taxane or taxane derivative, an aromatase inhibitor, an anthracycline, a microtubule targeting drug, a topoisomerase poison drug, an inhibitor of a molecular target or enzyme (e.g., a kinase inhibitor), a cytidine analogue drug or any chemotherapeutic, anti-neoplastic or anti-proliferative agent known in the art.

Exemplary alkylating agents include, but are not limited to, cyclophosphamide (Cytoxan; Neosar); chlorambucil (Leukeran); melphalan (Alkeran); carmustine (BiCNU); busulfan (Busulfex); lomustine (CeeNU); dacarbazine (DTIC-Dome); oxaliplatin (Eloxatin); carmustine (Gliadel); ifosfamide (Ifex); mechlorethamine (Mustargen); busulfan (Myleran); carboplatin (Paraplatin); cisplatin (CDDP; Platinol); temozolomide (Temodar); thiotepa (Thioplex); bendamustine (Treanda); or streptozocin (Zanosar).

Exemplary antibiotics include, but are not limited to, doxorubicin (Adriamycin); doxorubicin liposomal (Doxil); mitoxantrone (Novantrone); bleomycin (Blenoxane); daunorubicin (Cerubidine); daunorubicin liposomal (DaunoXome); dactinomycin (Cosmegen); epirubicin (Ellence); idarubicin (Idamycin); plicamycin (Mithracin); mitomycin (Mutamycin); pentostatin (Nipent); or valrubicin (Valstar).

Exemplary anti-metabolites include, but are not limited to, fluorouracil (Adrucil); capecitabine (Xeloda); hydroxyurea (Hydrea); mercaptopurine (Purinethol); pemetrexed (Alimta); fludarabine (Fludara); nelarabine (Arranon); cladribine (Cladribine Novaplus); clofarabine (Clolar); cytarabine (Cytosar-U); decitabine (Dacogen); cytarabine liposomal (DepoCyt); hydroxyurea (Droxia); pralatrexate (Folotyn); floxuridine (FUDR); gemcitabine (Gemzar); cladribine (Leustatin); fludarabine (Oforta); methotrexate (MTX; Rheumatrex); methotrexate (Trexall); thioguanine (Tabloid); TS-1 or cytarabine (Tarabine PFS).

Exemplary detoxifying agents include, but are not limited to, amifostine (Ethyol) or mesna (Mesnex).

Exemplary interferons include, but are not limited to, interferon alfa-2b (Intron A) or interferon alfa-2a (Roferon-A).

Exemplary polyclonal or monoclonal antibodies include, but are not limited to, trastuzumab (Herceptin); ofatumumab (Arzerra); bevacizumab (Avastin); rituximab (Rituxan); cetuximab (Erbitux); panitumumab (Vectibix); tositumomab/iodine 131 tositumomab (Bexxar); alemtuzumab (Campath); ibritumomab (Zevalin; In-I 11; Y-90 Zevalin); gemtuzumab (Mylotarg); eculizumab (Soliris); ordenosumab; ramucirumab (Cyramza) and olaratumab (Lartruvo).

Exemplary EGFR inhibitors include, but are not limited to, gefitinib (Iressa); lapatinib (Tykerb); cetuximab (Erbitux); erlotinib (Tarceva); panitumumab (Vectibix); PKI-166; canertinib (CI- 1033); matuzumab (Emd7200) or EKB-569.

Exemplary HER2 inhibitors include, but are not limited to, trastuzumab (Herceptin); lapatinib (Tykerb) or AC-480.

Histone Deacetylase Inhibitors include, but are not limited to, vorinostat (Zolinza).

Exemplary hormones include, but are not limited to, tamoxifen (Soltamox; Nolvadex); raloxifene (Evista); megestrol (Megace); leuprolide (Lupron; Lupron Depot; Eligard; Viadur) ; fulvestrant (Faslodex); letrozole (Femara); triptorelin (Trelstar LA; Trelstar Depot) ; exemestane (Aromasin) ; goserelin (Zoladex) ; bicalutamide (Casodex); anastrozole (Arimidex); fluoxymesterone (Androxy; Halotestin); medroxyprogesterone (Provera; Depo-Provera); estramustine (Emcyt); flutamide (Eulexin); toremifene (Fareston); degarelix (Firmagon); nilutamide (Nilandron); abarelix (Plenaxis); or testolactone (Teslac).

Exemplary mitotic inhibitors include, but are not limited to, paclitaxel (Taxol; Onxol; Abraxane); docetaxel (Taxotere); vincristine (Oncovin; Vincasar PFS); vinblastine (Velban); etoposide (Toposar; Etopophos; VePesid); teniposide (Vumon); ixabepilone (Ixempra); nocodazole; epothilone; vinorelbine (Navelbine); camptothecin (CPT); irinotecan (Camptosar); topotecan (Hycamtin); amsacrine or lamellarin D (LAM-D).

Exemplary MTOR inhibitors include, but are not limited to, everolimus (Afinitor) or temsirolimus (Torisel); rapamune, ridaforolimus; or AP23573.

Exemplary multi-kinase inhibitors include, but are not limited to, sorafenib (Nexavar); sunitinib (Sutent); BIBW 2992; E7080; Zd6474; PKC-412; motesanib; or AP24534.

Exemplary serine/threonine kinase inhibitors include, but are not limited to, ruboxistaurin; eril/easudil hydrochloride; flavopiridol; seliciclib (CYC202; Roscovitrine); SNS-032 (BMS-387032); Pkc412; bryostatin; KAI-9803;SF1126; VX-680; Azdl l52; Arry-142886 (AZD-6244); SCIO-469; GW681323; CC-401; CEP-1347 or PD 332991.

Exemplary tyrosine kinase inhibitors include, but are not limited to, erlotinib (Tarceva); gefitinib (Iressa); imatinib (Gleevec); sorafenib (Nexavar); sunitinib (Sutent); trastuzumab (Herceptin); bevacizumab (Avastin); rituximab (Rituxan); lapatinib (Tykerb); cetuximab (Erbitux); panitumumab (Vectibix); everolimus (Afinitor); alemtuzumab (Campath); gemtuzumab (Mylotarg); temsirolimus (Torisel); pazopanib (Votrient); dasatinib (Sprycel); nilotinib (Tasigna); vatalanib (Ptk787; ZK222584); CEP-701; SU5614; MLN518; XI,999; VX-322; Azd0530; BMS-354825; SKI-606 CP-690; AG-490; WHI-P154; WHI-P131; AC-220; or AMG888.

Exemplary VEGF/VEGFR inhibitors include, but are not limited to, bevacizumab (Avastin); sorafenib (Nexavar); sunitinib (Sutent); ranibizumab; ramucirumab (Cyramza) pegaptanib; or vandetinib.

Exemplary microtubule targeting drugs include, but are not limited to, paclitaxel, docetaxel, vincristin, vinblastin, nocodazole, epothilones and navelbine.

Exemplary topoisomerase poison drugs include, but are not limited to, teniposide, etoposide, adriamycin, camptothecin, daunorubicin, dactinomycin, mitoxantrone, amsacrine, epirubicin and idarubicin.

Exemplary taxanes or taxane derivatives include, but are not limited to, paclitaxel and docetaxol.

Exemplary general chemotherapeutic, anti-neoplastic, anti-proliferative agents include, but are not limited to, altretamine (Hexalen); isotretinoin (Accutane; Amnesteem; Clara vis; Sotret); tretinoin (Vesanoid); azacitidine (Vidaza); bortezomib (Velcade) asparaginase (Elspar); levamisole (Ergamisol); mitotane (Lysodren); procarbazine (Matulane); pegaspargase (Oncaspar); denileukin diftitox (Ontak); porfimer (Photofrin); aldesleukin (Proleukin); lenalidomide (Revlimid); bexarotene (Targretin); thalidomide (Thalomid); temsirolimus (Torisel); arsenic trioxide (Trisenox); verteporfin (Visudyne); mimosine (Leucenol); (1M tegafur - 0.4 M 5-chloro-2,4-dihydroxypyrimidine - 1 M potassium oxonate) or lovastatin.

In another aspect, the additional therapeutic agent can be a cytokine such as G-CSF (granulocyte colony stimulating factor). In another aspect, a composition the present disclosure, or a pharmaceutically acceptable salt, prodrug, metabolite, analog or derivative thereof, may be administered in combination with radiation therapy. Radiation therapy can also be administered in combination with a composition of the present disclosure and another chemotherapeutic agent described herein as part of a multiple agent therapy. In yet another aspect, a composition of the present disclosure, or a pharmaceutically acceptable salt, prodrug, metabolite, mimetic, analog or derivative thereof, may be administered in combination with standard chemotherapy combinations such as, but not restricted to, CMF (cyclophosphamide, methotrexate and 5-fluorouracil), CAF (cyclophosphamide, adriamycin and 5-fluorouracil), AC (adriamycin and cyclophosphamide), FEC (5-fluorouracil, epirubicin, and cyclophosphamide), ACT or ATC (adriamycin, cyclophosphamide, and paclitaxel), rituximab, Xeloda (capecitabine), Cisplatin (CDDP), Carboplatin, TS-1 (tegafur, gimestat and otastat potassium at a molar ratio of 1:0.4: 1), Camptothecin-11 (CPT-11, Irinotecan or Camptosar^{™}) or CMFP (cyclophosphamide, methotrexate, 5-fluorouracil and prednisone).

Exemplary kinase inhibitors include, but are not limited to, Bevacizumab (targets VEGF), BIBW 2992 (targets EGFR and Erb2), Cetuximab/Erbitux (targets Erbl), Imatinib/Gleevic (targets Bcr-Abl), Trastuzumab (targets Erb2), Gefitinib/Iressa (targets EGFR), Ranibizumab (targets VEGF), Pegaptanib (targets VEGF), Erlotinib/Tarceva (targets Erbl), Nilotinib (targets Bcr-Abl), Lapatinib (targets Erbl and Erb2/Her2), GW-572016/lapatinib ditosylate (targets HER2/Erb2), Panitumumab/Vectibix (targets EGFR), Vandetinib (targets RET/VEGFR), E7080 (multiple targets including RET and VEGFR), Herceptin (targets HER2/Erb2), PKI-166 (targets EGFR), Canertinib/CI-1033 (targets EGFR), Sunitinib/SU-11464/Sutent (targets EGFR and FLT3), Matuzumab/Emd7200 (targets EGFR), EKB-569 (targets EGFR), Zd6474 (targets EGFR and VEGFR), PKC-412 (targets VEGR and FLT3), Vatalanib/Ptk787/ZK222584 (targets VEGR), CEP-701 (targets FLT3), SU5614 (targets FLT3), MLN518 (targets FLT3), XL999 (targets FLT3), VX-322 (targets FLT3), Azd0530 (targets SRC), BMS-354825 (targets SRC), SKI-606 (targets SRC), CP-690 (targets JAK), AG-490 (targets JAK), WHI-P154 (targets JAK), WHI-P131 (targets JAK), sorafenib/Nexavar (targets RAF kinase, VEGFR- 1, VEGFR-2, VEGFR-3, PDGFR- β, KIT, FLT-3, and RET), Dasatinib/Sprycel (BCR/ABL and Src), AC-220 (targets Flt3), AC-480 (targets all HER proteins, "panHER"), Motesanib diphosphate (targets VEGF1-3, PDGFR, and c-kit), Denosumab (targets RANKL, inhibits SRC), AMG888 (targets HER3), and AP24534 (multiple targets including Flt3).

Exemplary serine/threonine kinase inhibitors include, but are not limited to, Rapamune (targets mTOR/FRAPl), Deforolimus (targets mTOR), Certican/Everolimus (targets mTOR/FRAPl), AP23573 (targets mTOR/FRAPl), Eril/Fasudil hydrochloride (targets RHO), Flavopiridol (targets CDK), Seliciclib/CYC202/Roscovitrine (targets CDK), SNS-032/BMS-387032 (targets CDK), Ruboxistaurin (targets PKC), Pkc412 (targets PKC), Bryostatin (targets PKC), KAI-9803 (targets PKC), SF1126 (targets PI3K), VX-680 (targets Aurora kinase), Azdl l52 (targets Aurora kinase), Arry-142886/AZD-6244 (targets MAP/MEK), SCIO-469 (targets MAP/MEK), GW681323 (targets MAP/MEK), CC-401 (targets JNK), CEP-1347 (targets JNK), and PD 332991 (targets CDK).

In particular embodiments, the methods further comprise administering an EGFR inhibitor to the subject in combination with the therapeutic agent (e.g., RRx-001 or a pharmaceutically acceptable salt thereof). In certain embodiments, the EGFR inhibitor is erlotinib or a pharmaceutically acceptable salt thereof. In certain embodiments, the EGFR inhibitor is erlotinib hydrochloride. In certain embodiments, the EGFR inhibitor comprises erlotinib.

The therapeutic method may be characterized according to the dose of erlotinib administered to the subject. For example, in certain embodiments, a daily dose of at least 500 mg of erlotinib is administered to the subject on any day on which erlotinib is administered to the subject. In certain embodiments, a daily dose of at least 1000 mg of erlotinib is administered to the subject on any day on which erlotinib is administered to the subject. In certain embodiments, a daily dose of at least 2000 mg of erlotinib is administered to the subject on any day on which erlotinib is administered to the subject. In certain other embodiments, a daily dose in the range of about 1,000 mg to about 3,000 mg of erlotinib is administered to the subject on any day on which erlotinib is administered to the subject. In certain embodiments, a daily dose in the range of about 1,500 mg to about 2,500 mg of erlotinib is administered to the subject on any day on which erlotinib is administered to the subject. In certain embodiments, a daily dose in the range of about 1,800 mg to about 2,200 mg of erlotinib is administered to the subject on any day on which erlotinib is administered to the subject. In certain embodiments, a daily dose of about 2,000 mg of erlotinib is administered to the subject on any day on which erlotinib is administered to the subj ect.

### Inorganic Nitrite Salt

In certain embodiments, the methods provided herein further comprise administering to the subject an inorganic nitrite salt in combination with the therapeutic agent (*e*.*g*., RRx-001 or a pharmaceutically acceptable salt thereof). In certain embodiments, the inorganic nitrite salt is an alkali metal nitrite. In certain embodiments, the inorganic nitrite salt is sodium nitrite.

In certain embodiments, the inorganic nitrite salt is administered before administering the first therapeutic agent to the subject, concurrently while administering the first therapeutic agent to the subject, after administering the first therapeutic agent to the subject, and/or each of the foregoing.

In certain embodiments, the inorganic nitrite salt is administered before the subject is exposed to radiation, concurrently while the subject is exposed to radiation, and/or after the subject has been exposed to radiation.

### Treatment of Biological Material with the Therapeutic Agent

In certain embodiments, the methods comprise treatment of biological materials, such as isolated cells (*e.g.*, blood cells), tissues, and organs, with the therapeutic agent (*e.g.*, RRx-001 or a pharmaceutically acceptable salt thereof). In some embodiments, the methods may be characterized according to the timing for exposing the biological material to the therapeutic agent. For example, in certain embodiments, the biological material is exposed to at least one dose of the therapeutic agent prior to exposure to the radiation. In certain embodiments, the biological material is exposed to at least one dose of the therapeutic agent within 1 day prior to exposure to the radiation. In certain embodiments, the biological material is exposed to at least one dose of the therapeutic agent within 12 hours prior to exposure to the radiation. In certain embodiments, the biological material is exposed to at least one dose of the therapeutic agent at least 6 hours prior to exposure to the radiation.

In certain other embodiments, the biological material is first exposed to a dose of the therapeutic agent during exposure to the radiation or after exposure to the radiation has ceased.

In certain non-claimed embodiments, the biological material is first exposed to a dose of the therapeutic agent during exposure to the radiation. In certain other embodiments of the invention, the biological material is first exposed to a dose of the therapeutic agent after exposure to the radiation has ceased. In certain embodiments, the biological material is first exposed to a dose of the therapeutic agent within 1 day after exposure to the radiation has ceased. In certain embodiments, the biological material is first exposed to a dose of the therapeutic agent within 12, 6, 3, or 2 hours after exposure to the radiation has ceased. In certain embodiments, the biological material is first exposed to a dose of the therapeutic agent within 1 hour after exposure to the radiation has ceased.

The method may be characterized according to the frequency of exposing the biological material to the therapeutic agent. For example, in certain embodiments, the biological material is exposed to the therapeutic agent no more frequently than once per week. In certain embodiments, the biological material is exposed to the therapeutic agent once per week for at least two weeks. In certain other embodiments, the biological material is exposed to the therapeutic agent at least once per week. In certain embodiments, the biological material is exposed to the therapeutic agent at least twice per week. In certain embodiments, the biological material is exposed to the therapeutic agent at least once per two days. In certain embodiments, the biological material is exposed to the therapeutic agent at least once per day. In certain embodiments, the biological material is exposed to the therapeutic agent at least twice per day.

In certain embodiments, the method further comprises exposing the biological material to an inorganic nitrite salt. In certain embodiments, the inorganic nitrite salt is an alkali metal nitrite. In certain embodiments, the inorganic nitrite salt is sodium nitrite. In certain embodiments, the inorganic nitrite salt is administered before administering the first therapeutic agent, concurrently while administering the first therapeutic agent, after administering the first therapeutic agent, and/or each of the foregoing.

In certain embodiments, the inorganic nitrite salt is administered before the biological material is exposed to radiation, concurrently while the biological material is exposed to radiation, and/or after the biological material has been exposed to radiation.

### Pharmaceutical Compositions

As indicated above, the present disclosure provides pharmaceutical compositions, which comprise an amount of one or more of the compounds described above, formulated together with one or more pharmaceutically acceptable carriers (additives) and/or diluents. The pharmaceutical compositions may be specially formulated for administration in solid or liquid form, including those adapted for the following: (1) oral administration, for example, drenches (aqueous or non-aqueous solutions or suspensions), tablets, *e*.*g*., those targeted for buccal, sublingual, and systemic absorption, boluses, powders, granules, pastes for application to the tongue; (2) parenteral administration, for example, by subcutaneous, intramuscular, intravenous or epidural injection as, for example, a sterile solution or suspension, or sustained-release formulation; (3) topical application, for example, as a cream, ointment, or a controlled-release patch or spray applied to the skin; (4) intravaginally or intrarectally, for example, as a pessary, cream or foam; (5) sublingually; (6) ocularly; (7) transdermally; or (8) nasally.

The phrase "therapeutically-effective amount" as used herein means that amount of a compound, material, or composition comprising a compound of the present disclosure which is effective for producing some desired therapeutic effect in at least a sub-population of cells in an animal at a reasonable benefit/risk ratio applicable to any medical treatment.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically-acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Formulations of the present disclosure include those suitable for oral, nasal, topical (including buccal and sublingual), rectal, vaginal and/or parenteral administration. The formulations may conveniently be presented in unit dosage form and may be prepared by any methods well known in the art of pharmacy. The amount of active ingredient which can be combined with a carrier material to produce a single dosage form will vary depending upon the host being treated, the particular mode of administration. The amount of active ingredient which can be combined with a carrier material to produce a single dosage form will generally be that amount of the compound which produces a therapeutic effect. Generally, out of one hundred percent, this amount will range from about 0.1 percent to about ninety-nine percent of active ingredient, preferably from about 5 percent to about 70 percent, most preferably from about 10 percent to about 30 percent.

In certain embodiments, a formulation of the present disclosure comprises an excipient selected from the group consisting of cyclodextrins, celluloses, liposomes, micelle forming agents, *e.g.*, bile acids, and polymeric carriers, *e*.*g*., polyesters and polyanhydrides; and a compound of the present disclosure. In certain embodiments, an aforementioned formulation renders orally bioavailable a compound of the present disclosure.

Methods of preparing these formulations or compositions include the step of bringing into association a compound of the present disclosure with the carrier and, optionally, one or more accessory ingredients. In general, the formulations are prepared by uniformly and intimately bringing into association a compound of the present disclosure with liquid carriers, or finely divided solid carriers, or both, and then, if necessary, shaping the product.

Formulations of the disclosure suitable for oral administration may be in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a compound of the present disclosure as an active ingredient. A compound of the present disclosure may also be administered as a bolus, electuary or paste.

In solid dosage forms of the disclosure for oral administration (capsules, tablets, pills, dragees, powders, granules, trouches and the like), the active ingredient is mixed with one or more pharmaceutically-acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds and surfactants, such as poloxamer and sodium lauryl sulfate; (7) wetting agents, such as, for example, cetyl alcohol, glycerol monostearate, and non-ionic surfactants; (8) absorbents, such as kaolin and bentonite clay; (9) lubricants, such as talc, calcium stearate, magnesium stearate, solid polyethylene glycols, sodium lauryl sulfate, zinc stearate, sodium stearate, stearic acid, and mixtures thereof; (10) coloring agents; and (11) controlled release agents such as crospovidone or ethyl cellulose.

In the case of capsules, tablets and pills, the pharmaceutical compositions may also comprise buffering agents. Solid compositions of a similar type may also be employed as fillers in soft and hard-shelled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered compound moistened with an inert liquid diluent.

The tablets, and other solid dosage forms of the pharmaceutical compositions of the present disclosure, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be formulated for rapid release, *e*.*g*., freeze-dried.

They may be sterilized by, for example, filtration through a bacteria-retaining filter, or by incorporating sterilizing agents in the form of sterile solid compositions which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions which can be used include polymeric substances and waxes. The active ingredient can also be in micro-encapsulated form, if appropriate, with one or more of the above-described excipients.

Liquid dosage forms for oral administration of the compounds of the disclosure include pharmaceutically acceptable emulsions, microemulsions, solutions, suspensions, syrups and elixirs. In addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate,

benzyl alcohol, benzyl benzoate, propylene glycol, 1,3-butylene glycol, oils (I particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, coloring, perfuming and preservative agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Formulations of the pharmaceutical compositions of the disclosure for rectal or vaginal administration may be presented as a suppository, which may be prepared by mixing one or more compounds of the disclosure with one or more suitable nonirritating excipients or carriers comprising, for example, cocoa butter, polyethylene glycol, a suppository wax or a salicylate, and which is solid at room temperature, but liquid at body temperature and, therefore, will melt in the rectum or vaginal cavity and release the active compound.

Formulations of the present disclosure which are suitable for vaginal administration also include pessaries, tampons, creams, gels, pastes, foams or spray formulations containing such carriers as are known in the art to be appropriate.

Dosage forms for the topical or transdermal administration of a compound of this disclosure include powders, sprays, ointments, pastes, creams, lotions, gels, solutions, patches and inhalants. The active compound may be mixed under sterile conditions with a pharmaceutically-acceptable carrier, and with any preservatives, buffers, or propellants which may be required.

The ointments, pastes, creams and gels may contain, in addition to an active compound of this disclosure, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to a compound of this disclosure, excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

Transdermal patches have the added advantage of providing controlled delivery of a compound of the present disclosure to the body. Such dosage forms can be made by dissolving or dispersing the compound in the proper medium. Absorption enhancers can also be used to increase the flux of the compound across the skin. The rate of such flux can be controlled by either providing a rate controlling membrane or dispersing the compound in a polymer matrix or gel.

Ophthalmic formulations, eye ointments, powders, solutions and the like, are also contemplated as being within the scope of this invention.

Pharmaceutical compositions of this disclosure suitable for parenteral administration comprise one or more compounds of the disclosure in combination with one or more pharmaceutically-acceptable sterile isotonic aqueous or nonaqueous solutions, dispersions, suspensions or emulsions, or sterile powders which may be reconstituted into sterile injectable solutions or dispersions just prior to use, which may contain sugars, alcohols, antioxidants, buffers, bacteriostats, solutes which render the formulation isotonic with the blood of the intended recipient or suspending or thickening agents.

Examples of suitable aqueous and nonaqueous carriers which may be employed in the pharmaceutical compositions of the disclosure include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms upon the subject compounds may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

In some cases, in order to prolong the effect of a drug, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution which, in turn, may depend upon crystal size and crystalline form. Alternatively, delayed absorption of a parenterally-administered drug form is accomplished by dissolving or suspending the drug in an oil vehicle.

Injectable depot forms are made by forming microencapsule matrices of the subject compounds in biodegradable polymers such as polylactide-polyglycolide. Depending on the ratio of drug to polymer, and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissue.

When the compounds of the present disclosure are administered as pharmaceuticals, to humans and animals, they can be given per se or as a pharmaceutical composition containing, for example, 0.1 to 99% (more preferably, 10 to 30%) of active ingredient in combination with a pharmaceutically acceptable carrier.

The preparations of the present disclosure may be given orally, parenterally, topically, or rectally. They are of course given in forms suitable for each administration route. For example, they are administered in tablets or capsule form, by injection, inhalation, eye lotion, ointment, suppository, etc. administration by injection, infusion or inhalation; topical by lotion or ointment; and rectal by suppositories. Oral administrations are preferred.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal and intrasternal injection and infusion.

The phrases "systemic administration," "administered systemically," "peripheral administration" and "administered peripherally" as used herein mean the administration of a compound, drug or other material other than directly into the central nervous system, such that it enters the patient's system and, thus, is subject to metabolism and other like processes, for example, subcutaneous administration.

These compounds may be administered to humans and other animals for therapy by any suitable route of administration, including orally, nasally, as by, for example, a spray, rectally, intravaginally, parenterally, intracisternally and topically, as by powders, ointments or drops, including buccally and sublingually.

Regardless of the route of administration selected, the compounds of the present disclosure, which may be used in a suitable hydrated form, and/or the pharmaceutical compositions of the present disclosure, are formulated into pharmaceutically-acceptable dosage forms by conventional methods known to those of skill in the art.

Actual dosage levels of the active ingredients in the pharmaceutical compositions of this disclosure may be varied so as to obtain an amount of the active ingredient which is effective to achieve the desired therapeutic response for a particular patient, composition, and mode of administration, without being toxic to the patient.

The selected dosage level will depend upon a variety of factors including the activity of the particular compound of the present disclosure employed, or the ester, salt or amide thereof, the route of administration, the time of administration, the rate of excretion or metabolism of the particular compound being employed, the rate and extent of absorption, the duration of the treatment, other drugs, compounds and/or materials used in combination with the particular compound employed, the age, sex, weight, condition, general health and prior medical history of the patient being treated, and like factors well known in the medical arts.

A physician or veterinarian having ordinary skill in the art can readily determine and prescribe the effective amount of the pharmaceutical composition required. For example, the physician or veterinarian could start doses of the compounds of the disclosure employed in the pharmaceutical composition at levels lower than that required in order to achieve the desired therapeutic effect and gradually increase the dosage until the desired effect is achieved.

In general, a suitable daily dose of a compound of the disclosure will be that amount of the compound which is the lowest dose effective to produce a therapeutic effect. Such an effective dose will generally depend upon the factors described above. Preferably, the compounds are administered at about 0.01 mg/kg to about 200 mg/kg, more preferably at about 0.1 mg/kg to about 100 mg/kg, even more preferably at about 0.5 mg/kg to about 50 mg/kg.

When the compounds described herein are co-administered with another agent (e.g., an additional radioprotective agent), the effective amount may be less than when the agent is used alone.

If desired, the effective daily dose of the active compound may be administered as two, three, four, five, six or more sub-doses administered separately at appropriate intervals throughout the day, optionally, in unit dosage forms. Preferred dosing is one administration per day.

### Kits for Use in Protecting Against and Reducing Effects of Radiation

Another aspect of the disclosure provides a kit for protecting against or reducing the effects of radiation. The kit comprises (i) a therapeutic agent selected from the group consisting of RRx-001 or a pharmaceutically acceptable salt thereof and (ii) instructions for protecting against, or reducing the effects of, radiation according to procedures described herein. In certain embodiments, the kit further comprises (iii) a local analgesic agent, such as lidocaine hydrochloride.

### EXAMPLES

### Example 1 - Effect of RRx-001 on Survival Following Lethal Irradiation

In this Example, the effects of systemic administration of RRx-001 on survival in response to a lethal dose of radiation was assayed in mice. CD2F1 male mice 9.5 - 11 weeks old were administered a single dose of RRx-001 by intraperitoneal (IP) injection 24 hours prior to a lethal radiation dose. 24 mice received 10 mg/kg RRx-001 (formulated in 5% DMSO in sterile H₂O) and 24 mice received the vehicle control (5% DMSO in sterile H₂O only).

The mice were subjected to total body irradiation (TBI) with 9.35 Gy (LD70/30) at 0.6 Gy/min using High-level Cobalt-60. Unanesthetized mice were placed in well-ventilated Plexiglas restrainers and irradiated bilaterally. Sham-irradiated mice were also placed in identical Plexiglas restrainers and kept in a room shielded from irradiation at the same time. In each experiment, the dose to the abdominal cores of the animals was delivered at a dose rate of approximately 0.6 Gy/min. Dosimetry was performed prior to the irradiation of the animals using the highly accurate alanine/electron spin resonance (ESR) dosimetry system (American Society for Testing and Materials, Standard E 1607) to measure dose rates (to water) in the cores of acrylic mouse phantoms, which were located in the compartments of the exposure rack. A calibration curve based on standard alanine calibration dosimeters provided by the National Institute of Standards and Technology (NIST, Gaithersburg, MD) was used to measure the doses. The accuracy of the dose rate calibrations has been verified several times using the services of the National Physics Laboratory (UK National Standards Laboratory, London, UK) and the M.D. Anderson Cancer Center (Houston, TX). The corrections applied to the measured dose rates in the phantoms were for a small difference in the Co-60 energy between the mass energy-absorption coefficients for soft tissue and water, as well as source decay. The radiation field was uniform within ± 1.2%.

Mice were monitored at least twice a day for 30 days post-irradiation. During the critical period (days 10 - 20), mice were monitored at least three times a day with no more than 10 hours between observations. Mice displaying any signs of discomfort received food in their cage as a wet mash. Mice displaying overt dyspnea, weight loss, lethargy, or other markers of moribundity and appearing to be in distress were humanely euthanized in a separate room using carbon dioxide gas followed by cervical dislocation after breathing stopped as a confirmatory method of euthanasia. This experiment was repeated for a total of n=24 mice per group.

Survival curves were estimated using the Kaplan-Meier method and were compared using a two-sided log-rank test at the 0.05 significance level. P-values were considered statistically significant if less than 0.05.

Survival improvement in favor of pretreatment with one dose of 10 mg/kg RRx-001 over vehicle control irradiated mice was highly significant with an approximate 33.4% reduction in the 30-day death risk (FIG. 1A). Time to death data depicting the 30-day survival is shown in FIG. 1B. A scatterplot of the survival times by treatment group show the means ± standard errors are 20.2 ± 1.6 and 27.2 ± 1.1 for vehicle and RRx-001 groups, respectively. Therefore, 10 mg/kg RRx-001 administered 24 hours prior to a lethal TBI dose not only significantly increases survival by 33.4% but also significantly increases the mean survival time by 7 days compared to the vehicle control.

### Example 2 - Effect of RRx-001 on Hematopoietic Recovery Following Irradiation

To determine the pathophysiological effects of RRx-001 on hematopoietic protection in mice, CD2F1 male mice were treated with 10 mg/kg RRx-001 or the vehicle control 24 hours prior to a sublethal dose of TBI (7 Gy at 0.6 Gy/min using High-level Cobalt-60) or sham irradiation (day 0) according to the table below.

| Group | Treatment | 2 days | 7 days | 14 days | 21 days | 28 days | Total mice |
|---|---|---|---|---|---|---|---|
| Vehicle Control | Sham + vehicle | 6 | 6 | 6 | 6 | 6 | 30 |
| Radiation Control | TBI + vehicle | 6 | 6 | 6 | 6 | 6 | 30 |
| Treatment Control | Sham + RRx-001 | 6 | 6 | 6 | 6 | 6 | 30 |
| Radiation Experimental | TBI + RRx-001 | 6 | 6 | 6 | 6 | 6 | 30 |

CD2F1 male mice (n=3/group) were divided into 4 experimental groups: 1) irradiation + vehicle, 2) irradiation + RRx-001, 3) sham-irradiation + vehicle and 4) sham-irradiation + RRx-001. Either 10 mg/kg RRx-001 or the vehicle control were IP injected 24 hours prior to either irradiation or sham-irradiation (day 0). On days 2, 7, 14, 21, and 28 post-irradiation (day 0) mice were humanely euthanized. Blood, bone marrow, and sternebrae were then collected. This experiment was performed in duplicate for a total of n=6 mice/group/time point.

Post-irradiation whole blood was obtained by terminal cardiocentesis. Blood samples were immediately transferred into EDTA tubes (Sarstedt Inc., Newton, NC) and gently rotated until the time of analysis. The tubes were analyzed for a complete blood count with differential and reticulocytes using the ADVIA 2120 (Siemens Medical Solutions Diagnostics, Dublin, Ireland), and Microsoft software version 5.9 (Microsoft Corp., Redmond, WA) to generate the data.

Sternebrae from euthanized mice (n = 6/group/time point) were collected on days 2, 7, 14, 21 and 28 post-irradiation. Sternebrae were fixed in 10% zinc-buffered formalin for at least 24 hours and up to 7 days. Fixed sternebrae were decalcified for 3 hr in 12-18% sodium EDTA (pH 7.4-7.5) and specimens dehydrated using graded ethanol concentrations and embedded in paraffin. Longitudinal 4 µm sections were stained with regular hematoxylin and eosin. Two board-certified pathologist conducted histopathological evaluation of the samples. One of the pathologist scored all the samples blindly. Bone marrow was evaluated in situ within sternebrae and graded (Grade 1: < 10%; Grade 2: 11-30%; Grade 3: 31-60%; Grade 4: 61-89%; Grade 5: > 90%) for total cellularity. Megakaryocytes were also quantified based on the average per 10 high power fields (HPF) at 400× magnification using a BX43 or BX53 microscope (Olympus, Minneapolis, MN). Images were captured with an Olympus DP22 camera and imported into Olympus Cellsens Standard software for review.

Blood parameters were compared between treatment groups using an analysis of variance (ANOVA). The Wilcoxon test was also used for sensitivity and to potentially address data departures from normality. A longitudinal mixed model repeated measures was also implemented to provide a more complete data analysis of the sham-irradiated treatment groups' difference in overall time profile mean based on the blood parameters. Bone marrow data (megakaryocytes and grade) statistical analysis was carried out using a parametric test consisting of a general linear model analysis of variance (ANOVA with factors consisting of treatment group and pathologist) and the Kruskal-Wallis nonparametric test. The statistical data analysis was carried out using R software (Version 3.4.3, 2016) and the graphs made using GraphPad Prism version 7.03 (GraphPad Software, La Jolla, CA).

To determine the effect of RRx-001 on bone marrow, a histopathological analysis of bone marrow sternebrae was performed and the cellularity, as reported by grade (grade 1: ≤10%; grade 2: 11-30%; grade 3: 31-60%; grade 4: 61-89%; grade 5: ≥90% cellularity), and megakaryocyte numbers (averaged per 10 high-powered fields; HPF) were ascertained by two pathologists, one of which scored all the samples blindly (TAS, WEC). In determining significance for grade of cellularity and average number of megakaryocytes per 10 HPF, the differences between pathologists and the interaction between treatment and pathologist were not significantly different.

The overall cellularity of the bone marrow in both the sham-irradiated RRx-001- and vehicle-treated groups never dropped below 90% during the duration of the study and therefore maintained a grade of 5 (FIG. 2). Figure 3A-B show representative normal bone marrow morphology and cellularity. As expected after irradiation, both the RRx-001- and vehicle-treated groups had a massive loss in bone marrow cellularity (grade 1). By day 7 a slight increase in cellularity was observed by the pathologists in the RRx-001-treated mice compared to the vehicle control. As shown in FIG. 2, pretreatment with RRx-001 significantly accelerated hematopoietic recovery as determined by the grade of bone marrow cellularity compared with control on day 14. The irradiated vehicle-treated group showed a significant loss of bone marrow cellularity with an increase in infiltration by adipocytes compared to the irradiated RRx-001-treated group on day 14 where significant recovery of bone marrow cellularity was observed (FIG. 5).

The number of megakaryocytes in the sham-irradiated RRx-001 group was significantly higher than the vehicle control on day 14 (FIG. 3). In both of the irradiated groups, the number of megakaryocytes was reduced on day 2 and severely depleted by day 7. The irradiated RRx-001 group shows a steady increase in the number of megakaryocytes between days 7 - 28. Interestingly, the irradiated vehicle-treated group had a significant jump in the number of megakaryocytes between days 14 - 21 before decreasing to return to the same megakaryocyte numbers as the irradiated RRx-001-treated group on day 28 (FIG. 3).

RRx-001 treatment also produced a significant increase in white blood cells and red blood cell production in the irradiated mice compared to the irradiated vehicle control (FIG. 4). A longitudinal mixed model repeated measures analysis comparing the difference in the overall mean of platelets over time revealed a statistically significant difference in the least-square means in favor of RRx-001-treatment (p = 0.01). The standard error of mean for the sham-irradiated RRx-001-treated versus vehicle-treated platelets were 1067.73 (32.26) and 913.66 (52.22), respectively. No significant difference in white blood cells (WBC), absolute neutrophil count (ANC), absolute lymphocyte count (ALC), platelets (PLT), percent hematocrit (% HCT) and percent reticulocytes (% RETIC) for days 2 and 21 post-irradiation was observed when comparing RRx-001-treated mice to the vehicle control (FIG. 4). Both irradiated RRx-001-treated and vehicle-treated groups showed a decrease in red blood cells and hemoglobin below the sham-irradiated controls on days 7 and 14 before returning to control levels; however, the irradiated groups were not significantly different when compared to each other.

In both experimental groups, the treatment with sublethal doses of acute irradiation induced severe reticulocytopenia and leukopenia. Reticulocytopenia persisted up to day 7 after irradiation. By day 14 after irradiation, %RETIC in RRx-001 pretreated mice was significantly increased and returned to baseline levels compared to control mice. Though both irradiated groups returned or were higher than baseline levels by day 28, RRx-001-treated mice still had significantly increased %RETIC compared to the vehicle controls. WBC and ALC reached their nadir on day 7 in both irradiated groups; however by day 14, WBC and ALC counts were also significantly increased in the RRx-001-treated mice compared to the controls. In both the irradiated RRx-001- and control-treated mice, the ANC and PLT reached their lowest point on day 7 and stayed there through day 14. However, for both the ANC and PLT, the irradiated mice pretreated with RRx-001 were significantly increased on both days 7 and 14 when compared to the control. Although %HCT reached its nadir on day 14, the irradiated RRx-001 mice had significantly increased levels on day 14 compared to the irradiated control.

The blood work and bone marrow obtained from the sublethal irradiation study show a significant increase in bone marrow cellularity and white and red blood cell production on day 14 in the RRx-001 irradiated group compared to the irradiated vehicle control. This may provide enough protection to allow for recovery during this crucial time period when infection and sepsis can occur. Taken together, these experiments demonstrate that systemic administration of RRx-001 prior to total body irradiation significantly improves overall survival and bone marrow regeneration.

### Example 3 - Characterization of the Radioprotective Effects of RRx-001

Antioxidant response element (ARE) genes such as heme oxygenase 1 (HO-1), NAD(P)H Dehydrogenase [Quinone] 1 (NQO-1) and Superoxide Dismutase (SOD) are involved in the detoxification and elimination of reactive oxidants. This example was designed to show that *in vitro* treatment with RRx-001 induces mild oxidative stress which increases Antioxidant Response Element (ARE) proteins in human normal bone marrow mesenchymal stem cells (hMSC), macrophages and their precursor monocyte cells. An exemplary treatment scheme is provided in FIG. 7.

Expression of ARE proteins was assayed in hMSC, macrophages, and monocytes following *in vitro* irradiation of the cells, which were pretreated with RRx-001 or vehicle. Protein expression was assayed by Western blotting and protein quantification.

hMSC were treated for 16 hours with 5 mM RRx-001 or the vehicle control (0.05% DMSO), irradiated at 10 Gy or sham-irradiated and the protein collected 8 or 24 hours post-sham or irradiation. Human monocytic leukemic THP-1 and U937 cells were differentiated into macrophages with 50 nM Phorbol 12-myristate 13-acetate (PMA) for 24 hours before treatment. Differentiated and non-differentiated cells were treated with 3 mM RRx-001 or 0.05% DMSO (vehicle control) for 16 hours prior to radiation. The cells were irradiated at 5 Gy or sham-irradiated and collected 4-8 hours later for both analysis. For each experiment, duplicates of each sample were run.

The activity of superoxide dismutase activity was also assayed in hMSC. hMSC were treated for 16 hours with 5 mM RRx-001 or vehicle, irradiated at 10 Gy or sham-irradiated and whole cell homogenate collected according to the manufacture's protocol. The assay measures the activity of all three forms of SOD. The amount of SOD activity (U/mL) was normalized to protein levels.

At 8 hours post-sham or irradiation, RRx-001 showed a significant increase in HO-1 expression (22 - 26 fold) in both the sham and irradiated hMSC groups (FIG. 8). At 24 hours, the RRx-001-treated groups still had an increase in HO-1 expression; however the increase dropped to 2 - 3.5 fold. RRx-001 treatment slightly reduced NQO-1 in all groups. SOD1 and 2 showed a slight increase at 8 hours after RRx-001 treatment and 10 Gy irradiation. Superoxide Dismutase Activity did not increase 8 hours after 10 Gy irradiation in the RRx-001 treated group (FIG. 9).

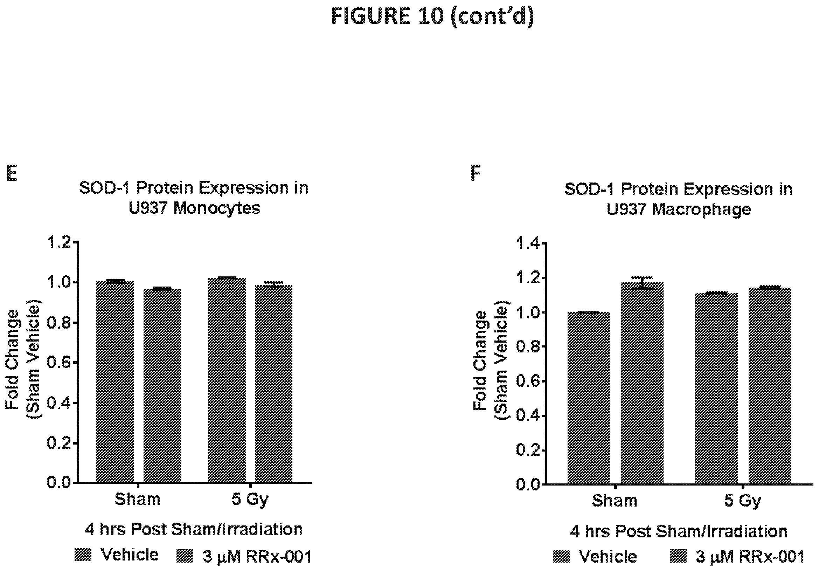

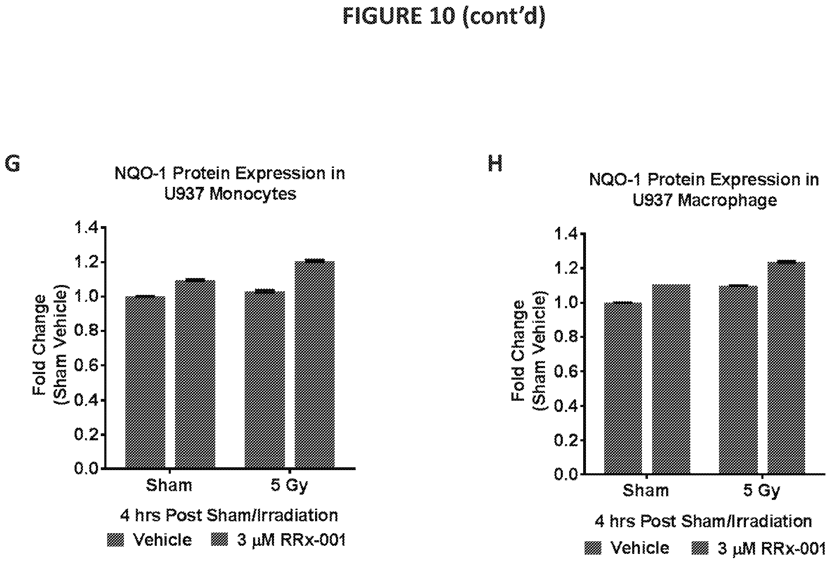

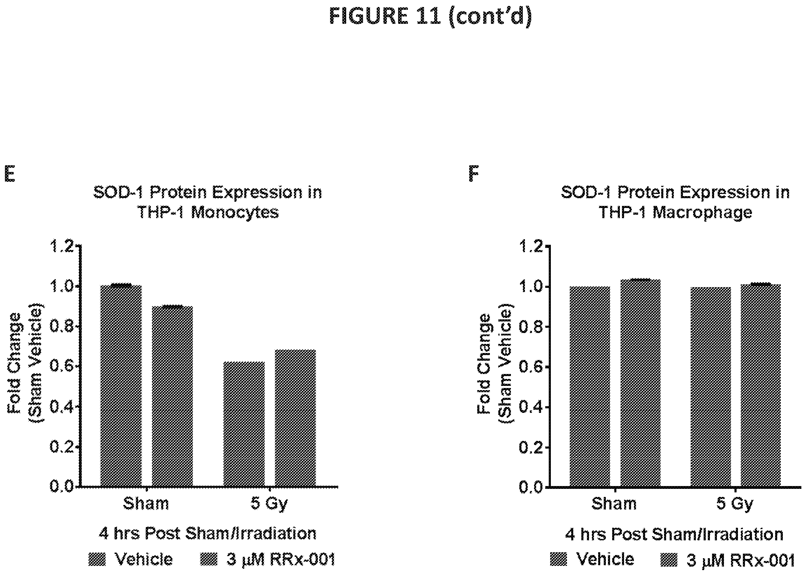

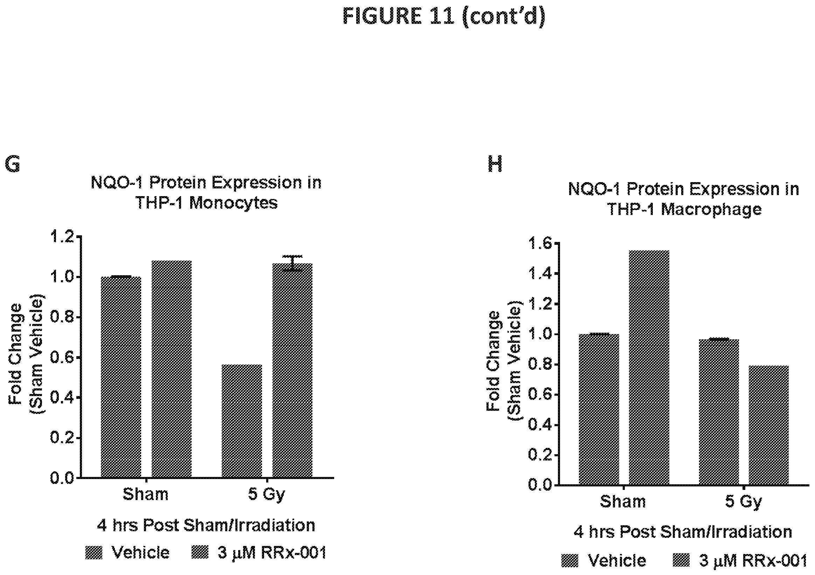

In the U937 macrophages, increased HO-1 production was observed after RRx-001 treatment in both the sham and irradiated groups; however no change in SOD-1 or NQO-1 was observed (FIG. 10). The results in the U937 monocytes were similar to those seen in the U937 macrophages. Overall, in both the U937 macrophage and monocyte fractions a significant increase in HO-1 was seen. A similar trend was also observed in the THP-1 monocytes; however, in the THP-1 macrophages no significant increase in HO-1 was seen (FIG. 11).

A cytokine array was employed to examine cytokine expression following *in vitro* irradiation of human monocytic leukemic THP-1 cells. THP-1 cells were differentiated into macrophages with 50 nM Phorbol 12-myristate 13-acetate (PMA) for 24 hours before treatment. Differentiated and non-differentiated cells were treated with 3 mM RRx-001 or 0.05% DMSO (vehicle control) for 16 hours prior to radiation. The cells were irradiated at 5 Gy or sham-irradiated and collected 4-8 hours later for both analysis. For the cytokine analysis, cell media was collected and blotted onto Proteome Profiler Human Cytokine Arrays (R&D Systems, Inc.) according to the manufacturer's protocol. The cytokine array showed a reduction in cytokines involved in inflammation in both the drug-treated sham and irradiated macrophage fraction as well as CCL5/RANTES induction in monocytes (FIG. 12).

The data suggest that RRx-001 may provide cellular protection from oxidative injury by increased HO-1 production in macrophage, monocytes, and mesenchymal stem cells. One potential mechanism is through the reduction in the pro-inflammatory chemokine IL-8 in macrophages and upregulation of CCL5/ RANTES in monocytes, which may enhance immune cell reprogramming. Without wishing to be bound by theory, the significant increase of HO-1 may protect the cells from apoptosis and DNA damage and increase their survival compared to cells that were not preconditioned with RRx-001.

### Example 4 -Assessment of RRx-001 for the Treatment of Oral Mucositis

In this example, the ability of RRx-001 to treat oral mucositis induced by acute radiation was assess in hamsters.

Fifty-six (56) male Syrian Golden Hamsters were used in the study. Mucositis was induced by administering an acute radiation dose of 40 Gy directed to the left buccal cheek pouch on Day 0 at a rate of 2-2.5 Gy/min. Mucositis was evaluated clinically starting on Day 6 and continuing on alternate days until Day 28. Hamsters reaching a mucositis severity score of 4 or higher received buprenorphine (0.5 mg/kg) SC twice a day for 48 hours or until score dropped below 4.

Dosing was scheduled as follows: for animals in Groups 1-4, animals were dosed with RRx-001 (1, 3, or 10 mg/kg) or vehicle (1:2 DMA:PEG400 vol:vol ratio) once a day (QD) on Days -4, -1, 1, 4, 7, 11, 14, 18, 21, and 25 via intraperitoneal (IP) administration; animals in Groups 5-7 were dosed QD with RRx-001 (1, 3, or 10 mg/kg) on Days -4, -1, 1, 8, 15, and 22.

| **Group #** | **# of Animals** | **Radiation (Day 0)** | **Treatment** | **Dose** | **Route** | **Dose Schedule*** | **Mucositis Evaluation (Q2D)** |
|---|---|---|---|---|---|---|---|
| 1 | 8 males | **40 Gy** | **Vehicle DMA-PEG** | --- | IP | QD | Day 6-28 |
| | | | (1:2 vol/vol ratio) | | | Days -4, -1,1, 4, 7, 11, 14, 18, 21 & 25 | |
| 2* | 8 males | **40 Gy** | RRx-001 | 10 mg/kg | IP | | |
| 3 | 8 males | **40 Gy** | RRx-001 | 3 mg/kg | IP | | |
| 4 | 8 males | **40 Gy** | RRx-001 | 1 mg/kg | IP | | |
| 5* | 8 males | **40 Gy** | RRx-001 | 10 mg/kg | IP | QD | |
| 6 | 8 males | **40 Gy** | RRx-001 | 3 mg/kg | IP | Days -4, -1, 1, 8, 15, 22 | |
| 7 | 8 males | **40 Gy** | RRx-001 | 1 mg/kg | IP | | |

Due to the presentation of adverse side effects following administration of the highest dose of RRx-001 (10mg/kg, Groups 2 and 5), dosing with this compound was discontinued for the remainder of the study after Day 1; however, the animals continued to be monitored and scored for the duration of the study. Dosing of all other groups continued as scheduled. Upon study conclusion, on Day 28, Animals were euthanized via CO₂ inhalation and death was confirmed by monitoring heartbeat in accordance with USDA guidelines. Animals steadily gained weight throughout the duration of the study, except for animals in Groups 2 and 5, which were characterized by lower weights than all other groups. For Groups 2 and 5, weight slowly recovered after cessation of dosing, and by study termination, weights had rebounded back to be in line with other groups on the study.

Mean daily mucositis scores are shown in FIG. 13. There was modest but significant enhancement of disease healing exhibited by animals treated with 10mg/kg in Group 2, though dosing was terminated after Day 1. All other treatment groups had mucositis scores that tracked fairly close to each other, and with Vehicle dosed controls.

The significance of differences observed between the control and treatment group was evaluated by comparing the days with mucositis scores ≥ 3 and < 3 between groups using chi-square analysis. Animals dosed with 10mg/kg and 1mg/kg RRx-001 displayed multiple days of significant improvement in mucositis scores compared to the Vehicle control group (FIG. 14). The percentage of animal days with a score of ≥ 3 in the Vehicle Group was 57.29%. The percentage of days with a score of ≥ 3 was statistically lower only for animals in Group 2 (dosed with 10mg/kg on Days -4, -1, 1) in comparison to the Vehicle Group (p<0.01). There were numerous days where animals dosed with RRx-001 had percent ulceration days that were lower (which can be interpreted as ameliorative of disease severity) in comparison to vehicle-dosed animals. For the 3 and 1mg/kg concentrations, twice weekly dosing appeared to provide more beneficial effects on percent days of ulceration than dosing one time per week (Groups 4 vs. Group 7). Interestingly, animals dosed with the 10mg/kg concentrations in both Groups 2 and 5, which were only dosed on Days -4, -1, and 1, had the best response in decreasing percent ulceration.

An analysis of the severity of mucositis was performed using the Mann-Whitney rank sum analysis to compare the visual mucositis scores for the treatment group to the Vehicle control group on each day of evaluation. The results of this analysis are shown in Table 5 and 6. In this analysis, 2 days of significant reduction in the mucositis score are generally required before it is regarded as meaningful. Animals dosed with 10mg/kg and 1mg/kg RX-001 displayed multiple days of significant improvement in mucositis scores compared to the Vehicle control group. A similar effect was observed for animals dosed 1X week, as shown in FIG 15.

The percentage of animals in each group with ulcerative mucositis at each day of evaluation is shown in FIG. 16. This evaluation was intended to clarify which days of treatment had its maximal impact on the course of ulcerative mucositis. There were numerous days where animals dosed with RRx-001 had percent ulceration days that were lower (which can be interpreted as ameliorative of disease severity) in comparison to vehicle-dosed animals. For 1mg/kg concentrations, twice weekly dosing appeared to provide more beneficial effects on percent days of ulceration than dosing one time per week (Groups 4 vs. Group 7). Interestingly, animals dosed with the 10mg/kg concentrations in both Groups 2 and 5, which were only dosed on Days -4, -1, and 1, had the best response in decreasing percent ulceration.

## Claims

1. A pharmaceutical composition comprising a therapeutic agent selected from the group consisting of RRx-001 and a pharmaceutically acceptable salt thereof, for use in a method for treating a subject in need of protection against radiation to thereby protect the subject against radiation, wherein at least one dose of the therapeutic agent is administered to the subject at least 6 hours prior to exposure to the radiation or after the exposure to the radiation has ceased.

2. The pharmaceutical composition for use of claim 1, wherein the administering achieves protection against radiation for a duration of at least 6 hours, at least 12 hours, at least 36 hours, at least 48 hours, at least 3 days, at least 4 days, at least 5 days, at least 6 days, at least 1 week, at least 2 weeks, at least 3 weeks, at least 4 weeks or longer.

3. A pharmaceutical composition comprising a therapeutic agent selected from the group consisting of RRx-001 and a pharmaceutically acceptable salt thereof, for use in a method of reducing radiation-exposure damage to a subject to thereby reduce radiation-exposure damage to the subject, wherein at least one dose of the therapeutic agent is administered to the subject at least 6 hours prior to exposure to the radiation or after the exposure to the radiation has ceased.

4. The pharmaceutical composition for use of claim 3, wherein the administering reduces radiation-exposure damage to the subject for a duration of at least 6 hours, at least 12 hours, at least 36 hours, at least 48 hours, at least 3 days, at least 4 days, at least 5 days, at least 6 days, at least 1 week, at least 2 weeks, at least 3 weeks, at least 4 weeks or longer.

5. The pharmaceutical composition for use of any one of claims 1-4, wherein (i) at least one dose of the therapeutic agent is administered to the subject within 12 hours, 24 hours, 36 hours, 48 hours, 3 days, 4 days, 5 days, 6 days, or 1 week prior to exposure to the radiation or within 48, 24, or 12 hours after exposure to the radiation has ceased; or (ii) the therapeutic agent is administered at a dosage that provides RRx-001 in an amount ranging from about 0.01 mg to about 500 mg, about 0.1 mg to about 200 mg, or about 0.5 mg to about 150 mg of RRx-001 on each day the therapeutic agent is administered to the subject.

6. The pharmaceutical composition for use of any one of claims 1-5, wherein
(I) the therapeutic agent is administered by a route selected from the group consisting of parenteral administration, oral administration and topical administration;
(II) (a) the therapeutic agent is administered intravenously to the subject, optionally wherein the therapeutic agent is administered intravenously to the subject as a single bolus injection, multiple injections, or infused over time and optionally, wherein the therapeutic agent is administered intravenously to the subject over a duration of at least thirty minutes;
(b) the therapeutic agent is administered by intraperitoneal injection to the subject, optionally, wherein the therapeutic agent is administered by intraperitoneal injection to the subject as a single bolus injection, multiple injections, or infused over time, and, optionally, wherein the therapeutic agent is administered by intraperitoneal injection to the subject over a duration of at least thirty minutes;
wherein (c) the therapeutic agent is administered by subcutaneous injection, optionally, wherein the therapeutic agent is administered by subcutaneous injection to the subject as a single bolus injection, multiple injections, or infused over time, and, optionally, wherein the therapeutic agent is administered by subcutaneous injection to the subject over a duration of at least 5 minutes; or
(d) wherein the therapeutic agent is administered subcutaneously to the subject via a pump device implanted in the subject that contains the therapeutic agent, optionally, wherein the pump device is an osmotic pump; or
(III) wherein the therapeutic agent is administered to the subject once per week, once per week for at least two weeks, twice per week, or twice per week for at least two weeks.

7. The pharmaceutical composition for use of any one of claims 1-6, wherein (I) the method further comprises, prior to administration of the therapeutic agent, administering to the subject (a) a pain-relieving agent, (b) a local analgesic agent to tissue in proximity to the site of administration of the therapeutic agent; or (II) the method further comprises administering to the subject (a) an EGFR inhibitor; or (b) an inorganic nitrite salt.

8. The pharmaceutical composition for use of claim 7, wherein (i) the pain-relieving agent is aspirin, a corticosteroid, or a non-steroidal anti-inflammatory agent; (ii) the local analgesic agent is selected from a caine analgesic, lidocaine, lidocaine hydrochloride, VanPen cream, NSAID, and acetaminophen, (iii) the EGFR inhibitor is administered to the subject according to a pulse-dosing schedule; (iv) the EGFR inhibitor is erlotinib or a pharmaceutically acceptable salt thereof; (v) the inorganic nitrite salt is an alkali metal nitrite or sodium nitrite.

9. The pharmaceutical composition for use of any one of claims 1-8, wherein the therapeutic agent is administered in proximity to tissue desired to be protected from radiation, optionally, wherein said tissue is bone marrow, skin, pulmonary tissue, thyroid tissue, gonadal tissue, tissue of the gastrointestinal tract, skeletal tissue, fetal tissue, or a combination thereof.

10. The pharmaceutical composition for use of any one of claims 1-9, wherein the subject is an adult human, pediatric human, animal, subject at risk of exposure to radiation from a nuclear emergency, subject receiving a radiation therapy, or subject suffering from or at risk of suffering from mucositis, optionally, wherein the radiation therapy is for the treatment of a cancer.

11. An *in vitro* method of protecting biological material from the damaging effects of radiation, comprising exposing said biological material to at least one dose of a therapeutic agent selected from the group consisting of RRx-001 and a pharmaceutically acceptable salt thereof at least 6 hours prior to exposure to radiation or after the exposure to the radiation has ceased, to thereby protect the biological material from the damaging effects of radiation.

12. The method of claim 11, wherein (i) the biological material is protected from the damaging effects of radiation for a duration of at least 6 hours, at least 12 hours, at least 36 hours, at least 48 hours, at least 3 days, at least 4 days, at least 5 days, at least 6 days, at least 1 week, at least 2 weeks, at least 3 weeks, at least 4 weeks or longer; (ii) the biological material is exposed to the at least one dose of the therapeutic agent within 24 hours prior to exposure to the radiation; (iii) the biological material is exposed to the therapeutic agent once per week; or (iv) the biological material is exposed to the therapeutic agent once per week for at least two weeks.

13. The method of any one of claims 11-12, wherein the biological material comprises an isolated cell, an isolated tissue or an isolated organ, or a blood cell.

14. The pharmaceutical composition for use of any one of claims 1-10 or the method of any one of claims 11-13, wherein (i) the radiation is ionizing radiation or ionizing radiation from sunlight, radioactive nuclei, or an explosive device; or (ii) the radiation comprises α-rays, β-rays, γ-rays, neutron radiation, x-rays, or a combination thereof.

15. The pharmaceutical composition for use of any one of claims 1-10 or the method of any one of claims 11-14, wherein the therapeutic agent is RRx-001.

## Patentansprüche

1. Pharmazeutische Zusammensetzung, die ein therapeutisches Mittel umfasst, ausgewählt aus der Gruppe bestehend aus RRx-001 und einem pharmazeutisch akzeptablen Salz davon, zur Verwendung in einem Verfahren zur Behandlung eines Subjekts, das einen Schutz gegen Strahlung benötigt, um dadurch das Subjekt gegen Strahlung zu schützen, wobei mindestens eine Dosis des therapeutischen Mittels dem Subjekt mindestens 6 Stunden vor der Strahlenexposition oder nachdem die Strahlenexposition beendet ist verabreicht wird.

2. Pharmazeutische Zusammensetzung zur Verwendung nach Anspruch 1, wobei die Verabreichung einen Schutz gegen Strahlung für eine Dauer von mindestens 6 Stunden, mindestens 12 Stunden, mindestens 36 Stunden, mindestens 48 Stunden, mindestens 3 Tage, mindestens 4 Tage, mindestens 5 Tage, mindestens 6 Tage, mindestens 1 Woche, mindestens 2 Wochen, mindestens 3 Wochen, mindestens 4 Wochen oder länger erreicht.

3. Pharmazeutische Zusammensetzung, die ein therapeutisches Mittel umfasst, ausgewählt aus der
Gruppe bestehend aus RRx-001 und einem pharmazeutisch akzeptablen Salz davon, zur Verwendung in einem Verfahren zur Verringerung von Schäden durch Strahlenexposition bei einem Subjekt, um dadurch Schäden durch Strahlenexposition bei dem Subjekt zu verringern, wobei mindestens eine Dosis des therapeutischen Mittels dem Subjekt mindestens 6 Stunden vor der Strahlenexposition oder nachdem die Strahlenexposition beendet ist verabreicht wird.

4. Pharmazeutische Zusammensetzung zur Verwendung nach Anspruch 3, wobei die Verabreichung Schäden durch Strahlenexposition beim Subjekt für eine Dauer von mindestens 6 Stunden, mindestens 12 Stunden, mindestens 36 Stunden, mindestens 48 Stunden, mindestens 3 Tage, mindestens 4 Tage, mindestens 5 Tage, mindestens 6 Tage, mindestens 1 Woche, mindestens 2 Wochen, mindestens 3 Wochen, mindestens 4 Wochen oder länger reduziert.

5. Pharmazeutische Zusammensetzung zur Verwendung nach einem der Ansprüche 1 bis 4, wobei (i) mindestens eine Dosis des therapeutischen Mittels dem Subjekt innerhalb von 12 Stunden, 24 Stunden, 36 Stunden, 48 Stunden, 3 Tagen, 4 Tagen, 5 Tagen, 6 Tagen oder 1 Woche vor der Strahlenexposition oder innerhalb von 48, 24 oder 12 Stunden nach Ende der Strahlenexposition verabreicht wird; oder (ii) das therapeutische Mittel in einer Dosierung verabreicht wird, die RRx-001 in einer Menge im Bereich von etwa 0,01 mg bis etwa 500 mg, etwa 0,1 mg bis etwa 200 mg oder etwa 0,5 mg bis etwa 150 mg RRx-001 an jedem Tag bereitstellt, an dem das therapeutische Mittel dem Subjekt verabreicht wird.

6. Pharmazeutische Zusammensetzung zur Verwendung nach einem der Ansprüche 1-5, wobei
(I) das therapeutische Mittel auf einem Weg verabreicht wird, der ausgewählt ist aus der Gruppe bestehend aus parenteraler Verabreichung, oraler Verabreichung und topischer Verabreichung;
(II) (a) das therapeutische Mittel dem Subjekt intravenös verabreicht wird, wobei optional das therapeutische Mittel dem Subjekt als einzelne Bolusinjektion, mehrere Injektionen oder als Infusion über einen Zeitraum verabreicht wird und wobei optional das therapeutische Mittel dem Subjekt intravenös über einen Zeitraum von mindestens 30 Minuten verabreicht wird;
(b) das therapeutische Mittel dem Subjekt durch intraperitoneale Injektion verabreicht wird, wobei optional das therapeutische Mittel dem Subjekt durch intraperitoneale Injektion als einzelne Bolusinjektion, mehrere Injektionen oder als Infusion über einen Zeitraum verabreicht wird, und, wobei optional das therapeutische Mittel dem Subjekt durch intraperitoneale Injektion über einen Zeitraum von mindestens dreißig Minuten verabreicht wird;
wobei (c) das therapeutische Mittel durch subkutane Injektion verabreicht wird, wobei optional das therapeutische Mittel durch subkutane Injektion an das Subjekt als einzelne Bolusinjektion, mehrere Injektionen oder als Infusion über einen Zeitraum verabreicht wird, und wobei optional das therapeutische Mittel dem Subjekt durch subkutane Injektion über eine Dauer von mindestens 5 Minuten verabreicht wird; oder
(d) wobei das therapeutische Mittel dem Subjekt subkutan über eine in das Subjekt implantierte Pumpvorrichtung, die das therapeutische Mittel enthält, verabreicht wird, wobei optional die Pumpvorrichtung eine osmotische Pumpe ist; oder
(III) wobei das therapeutische Mittel dem Subjekt einmal pro Woche, einmal pro Woche für mindestens zwei Wochen, zweimal pro Woche oder zweimal pro Woche für mindestens zwei Wochen verabreicht wird.

7. Pharmazeutische Zusammensetzung zur Verwendung nach einem der Ansprüche 1 bis 6, wobei (I) die Methode vor der Verabreichung des therapeutischen Mittels weiterhin die Verabreichung (a) eines schmerzlindernden Mittels, (b) eines lokalen analgetischen Mittels an das Gewebe in der Nähe der Verabreichungsstelle des therapeutischen Mittels, an das Subjekt umfasst; oder (II) die Methode weiterhin die Verabreichung (a) eines EGFR-Inhibitors; oder (b) eines anorganischen Nitritsalzes an das Subjekt umfasst.

8. Pharmazeutische Zusammensetzung zur Verwendung nach Anspruch 7, wobei (i) das schmerzlindernde Mittel Aspirin, ein Kortikosteroid oder ein nichtsteroidales anti-inflammatorisches Mittel ist; (ii) das lokale analgetische Mittel ausgewählt ist aus einem Cain-Analgetikum, Lidocain, Lidocainhydrochlorid, VanPen-Creme, NSAID und Acetaminophen, (iii) der EGFR-Inhibitor dem Subjekt nach einem Puls-Dosierungsschema verabreicht wird; (iv) der EGFR-Inhibitor Erlotinib oder ein pharmazeutisch akzeptables Salz davon ist; (v) das anorganische Nitritsalz ein Alkalimetallnitrit oder Natriumnitrit ist.

9. Pharmazeutische Zusammensetzung zur Verwendung nach einem der Ansprüche 1 bis 8, wobei das therapeutische Mittel in der Nähe von Gewebe verabreicht wird, das vor Strahlung geschützt werden soll, wobei das Gewebe optional Knochenmark, Haut, Lungengewebe, Schilddrüsengewebe, Gonadengewebe, Gewebe des Gastrointestinaltrakts, Skelettgewebe, fötales Gewebe oder eine Kombination davon ist.

10. Pharmazeutische Zusammensetzung zur Verwendung nach einem der Ansprüche 1 bis 9, wobei das Subjekt ein erwachsener Mensch, ein pädiatrischer Mensch, ein Tier, ein Subjekt mit dem Risiko einer Strahlenexposition eines nuklearen Notfalls, ein Subjekt, das eine Strahlentherapie erhält, oder ein Subjekt, das an Mukositis leidet oder das Risiko hat, daran zu leiden, ist, wobei optional die Strahlentherapie zur Behandlung von Krebs dient.

11. *In-vitro* Methode zum Schutz von biologischem Material vor den schädigenden Effekten von Strahlung, die das Aussetzen des biologischen Materials gegenüber mindestens einer Dosis eines therapeutischen Mittels, ausgewählt aus der Gruppe bestehend aus RRx-001 und einem pharmazeutisch akzeptablen Salz davon mindestens 6 Stunden vor der Strahlenexposition oder nachdem die Strahlenexposition beendet ist, umfasst, um dadurch das biologische Material vor den schädigenden Effekten der Strahlung zu schützen.

12. Methode nach Anspruch 11, wobei (i) das biologische Material für eine Dauer von mindestens 6 Stunden, mindestens 12 Stunden, mindestens 36 Stunden, mindestens 48 Stunden, mindestens 3 Tagen, mindestens 4 Tagen, mindestens 5 Tagen, mindestens 6 Tagen, mindestens 1 Woche, mindestens 2 Wochen, mindestens 3 Wochen, mindestens 4 Wochen oder länger vor den schädlichen Wirkungen der Strahlung geschützt wird; (ii) das biologische Material der mindestens einen Dosis des therapeutischen Mittels innerhalb von 24 Stunden vor der Strahlenexposition ausgesetzt wird; (iii) das biologische Material dem therapeutischen Mittel einmal pro Woche ausgesetzt wird; oder (iv) das biologische Material dem therapeutischen Mittel einmal pro Woche für mindestens zwei Wochen ausgesetzt wird.

13. Methode nach einem der Ansprüche 11 bis 12, wobei das biologische Material eine isolierte Zelle, ein isoliertes Gewebe oder ein isoliertes Organ oder eine Blutzelle umfasst.

14. Pharmazeutische Zusammensetzung zur Verwendung nach einem der Ansprüche 1-10 oder die Methode nach einem der Ansprüche 11-13, wobei (i) die Strahlung ionisierende Strahlung oder ionisierende Strahlung aus Sonnenlicht, radioaktiven Kernen oder einer Sprengvorrichtung ist; oder (ii) die Strahlung α-Strahlen, β-Strahlen, y-Strahlen, Neutronenstrahlung, Röntgenstrahlen oder eine Kombination davon umfasst.

15. Pharmazeutische Zusammensetzung zur Verwendung nach einem der Ansprüche 1-10 oder die Methode nach einem der Ansprüche 11-14, wobei das therapeutische Mittel RRx-001 ist.

## Revendications

1. Composition pharmaceutique comprenant un agent thérapeutique sélectionné parmi le groupe constitué de RRx-001 et d'un sel pharmaceutiquement acceptable de celui-ci, à utiliser dans un procédé de traitement d'un sujet qui a besoin d'une protection contre un rayonnement, dans le but de protéger ainsi le sujet contre un rayonnement, dans laquelle au moins une dose de l'agent thérapeutique est administrée au sujet au moins 6 heures avant l'exposition au rayonnement ou après que l'exposition au rayonnement a cessé.

2. Composition pharmaceutique pour une utilisation selon la revendication 1, dans laquelle l'administration permet d'obtenir une protection contre un rayonnement pendant une durée d'au moins 6 heures, d'au moins 12 heures, d'au moins 36 heures, d'au moins 48 heures, d'au moins 3 jours, d'au moins 4 jours, d'au moins 5 jours, d'au moins 6 jours, d'au moins 1 semaine, d'au moins 2 semaines, d'au moins 3 semaines, d'au moins 4 semaines, ou plus longtemps.

3. Composition pharmaceutique comprenant un agent thérapeutique sélectionné parmi le groupe constitué de RRx-001 et d'un sel pharmaceutiquement acceptable de celui-ci, à utiliser dans un procédé de réduction de dommages causés par une exposition à un rayonnement chez un sujet dans le but de réduire ainsi les dommages causés par une exposition à un rayonnement chez le sujet, dans lequel au moins une dose de l'agent thérapeutique est administrée au sujet au moins 6 heures avant l'exposition au rayonnement ou après que l'exposition au rayonnement a cessé.

4. Composition pharmaceutique pour une utilisation selon la revendication 3, dans laquelle l'administration réduit les dommages causés par une exposition à un rayonnement pendant une durée d'au moins 6 heures, d'au moins 12 heures, d'au moins 36 heures, d'au moins 48 heures, d'au moins 3 jours, d'au moins 4 jours, d'au moins 5 jours, d'au moins 6 jours, d'au moins 1 semaine, d'au moins 2 semaines, d'au moins 3 semaines, d'au moins 4 semaines, ou plus longtemps.

5. Composition pharmaceutique pour une utilisation selon l'une quelconque des revendications 1 à 4, dans laquelle (i) au moins une dose de l'agent thérapeutique est administrée au sujet dans les 12 heures, 24 heures, 36 heures, 48 heures, 3 jours, 4 jours, 5 jours, 6 jours ou 1 semaine avant l'exposition au rayonnement ou dans les 48, 24 ou 12 heures après que l'exposition au rayonnement a cessé ; ou (ii) l'agent thérapeutique est administré à une dose qui fournit du RRx-001 en une quantité comprise dans une plage allant d'environ 0,01 mg à environ 500 mg, d'environ 0,1 mg à environ 200 mg, ou d'environ 0,5 mg à environ 150 mg de RRx-001 chaque jour où l'agent thérapeutique est administré au sujet.

6. Composition pharmaceutique pour une utilisation selon l'une quelconque des revendications 1 à 5, dans laquelle
(I) l'agent thérapeutique est administré par une voie sélectionnée parmi le groupe constitué de l'administration parentérale, l'administration orale et l'administration topique ;
(II) (a) l'agent thérapeutique est administré au sujet par voie intraveineuse, optionnellement, dans laquelle l'agent thérapeutique est administré au sujet par voie intraveineuse sous la forme d'une injection en bolus unique, d'injections multiples ou d'une perfusion étalée dans le temps et, optionnellement, dans laquelle l'agent thérapeutique est administré au sujet par voie intraveineuse pendant une durée d'au moins trente minutes ;
(b) l'agent thérapeutique est administré au sujet par injection intrapéritonéale,
optionnellement, dans laquelle l'agent thérapeutique est administré au sujet par injection intrapéritonéale sous la forme d'une injection en bolus unique, d'injections multiples ou d'une perfusion étalée dans le temps, et, optionnellement, dans laquelle l'agent thérapeutique est administré au sujet par injection intrapéritonéale pendant une durée d'au moins trente minutes ;dans laquelle (c) l'agent thérapeutique est administré par injection sous-cutanée, optionnellement, dans laquelle l'agent thérapeutique est administré au sujet par injection sous-cutanée sous la forme d'une injection en bolus unique, d'injections multiples ou d'une perfusion étalée dans le temps, et, optionnellement, dans laquelle l'agent thérapeutique est administré au sujet par injection sous-cutanée pendant une durée d'au moins 5 minutes ; ou
(d) dans laquelle l'agent thérapeutique est administré au sujet par voie sous-cutanée par l'intermédiaire d'un dispositif de pompe implanté chez le sujet et qui contient l'agent thérapeutique, optionnellement, dans laquelle le dispositif de pompe est une pompe osmotique ; ou
(III) dans laquelle l'agent thérapeutique est administré au sujet une fois par semaine, une fois par semaine pendant au moins deux semaines, deux fois par semaine ou deux fois par semaine pendant au moins deux semaines.

7. Composition pharmaceutique pour une utilisation selon l'une quelconque des revendications 1 à 6, dans laquelle (I) le procédé comprend en outre, avant l'administration de l'agent thérapeutique, une administration au sujet (a) d'un agent analgésique, (b) d'un agent analgésique local à un tissu à proximité du site d'administration de l'agent thérapeutique ; ou (II) le procédé comprend en outre une administration au sujet (a) d'un inhibiteur de l'EGFR ; ou (b) d'un sel de nitrite inorganique.

8. Composition pharmaceutique pour une utilisation selon la revendication 7, dans laquelle (i) l'agent analgésique est l'aspirine, un corticostéroïde ou un agent anti-inflammatoire non stéroïdien ; (ii) l'agent analgésique local est sélectionné parmi un analgésique caïnique, la lidocaïne, le chlorhydrate de lidocaïne, la crème VanPen, un AINS et l'acétaminophène, (iii) l'inhibiteur de l'EGFR est administré au sujet suivant un schéma posologique à impulsions ; (iv) l'inhibiteur de l'EGFR est l'erlotinib ou un sel pharmaceutiquement acceptable de celui-ci ; (v) le sel de nitrite inorganique est un nitrite de métal alcalin ou un nitrite de sodium.

9. Composition pharmaceutique pour une utilisation selon l'une quelconque des revendications 1 à 8, dans laquelle l'agent thérapeutique est administré à proximité d'un tissu que l'on souhaite protéger contre un rayonnement, optionnellement, dans laquelle ledit tissu est la moelle osseuse, la peau, le tissu pulmonaire, le tissu thyroïdien, le tissu gonadique, le tissu du tractus gastro-intestinal, le tissu squelettique, le tissu foetal, ou une combinaison de ceux-ci.

10. Composition pharmaceutique pour une utilisation selon l'une quelconque des revendications 1 à 9, dans laquelle le sujet est un humain adulte, un humain pédiatrique, un animal, un sujet qui risque d'être exposé à un rayonnement provenant d'une urgence nucléaire, un sujet qui reçoit une radiothérapie ou un sujet qui souffre ou risque de souffrir d'une mucosité, optionnellement, dans laquelle la radiothérapie est destinée au traitement d'un cancer.

11. Procédé *in vitro* de protection d'un matériel biologique contre les effets néfastes d'un rayonnement, comprenant une exposition dudit matériel biologique au moins une dose d'un agent thérapeutique sélectionné parmi le groupe constitué de RRx-001 et d'un sel pharmaceutiquement acceptable de celui-ci au moins 6 heures avant l'exposition au rayonnement ou après que l'exposition au rayonnement a cessé, afin de protéger ainsi le matériel biologique contre les effets néfastes du rayonnement.

12. Procédé selon la revendication 11, dans lequel (i) le matériel biologique est protégé contre les effets néfastes du rayonnement pendant une durée d'au moins 6 heures, d'au moins 12 heures, d'au moins 36 heures, d'au moins 48 heures, d'au moins 3 jours, d'au moins 4 jours, d'au moins 5 jours, d'au moins 6 jours, d'au moins 1 semaine, d'au moins 2 semaines, d'au moins 3 semaines, d'au moins 4 semaines ou plus ; (ii) le matériel biologique est exposé à l'au moins une dose de l'agent thérapeutique dans les 24 heures qui précèdent l'exposition au rayonnement ; (iii) le matériel biologique est exposé à l'agent thérapeutique une fois par semaine ; ou (iv) le matériel biologique est exposé à l'agent thérapeutique une fois par semaine pendant au moins deux semaines.

13. Procédé selon l'une quelconque des revendications 11 à 12, dans lequel le matériel biologique comprend une cellule isolée, un tissu isolé ou un organe isolé, ou une cellule sanguine.

14. Composition pharmaceutique pour une utilisation selon l'une quelconque des revendications 1 à 10 ou procédé selon l'une quelconque des revendications 11 à 13, dans laquelle/lequel (i) le rayonnement est un rayonnement ionisant, ou un rayonnement ionisant provenant de la lumière du soleil, de noyaux radioactifs ou d'un dispositif explosif ; ou (ii) le rayonnement comprend des rayons α, des rayons β, des rayons γ, un rayonnement neutronique, des rayons X ou une combinaison de ceux-ci.

15. Composition pharmaceutique pour une utilisation selon l'une quelconque des revendications 1 à 10 ou procédé selon l'une quelconque des revendications 11 à 14, dans laquelle/lequel l'agent thérapeutique est le RRx-001.
